# EUROPEAN PATENT APPLICATION

(11) **EP 3 885 442 A1**
(43) Date of publication of application: **29.09.2021**
(21) Application number: 19887837.3
(22) Date of filing: 21.11.2019
(51) Int. Cl.: C12P 19/02, C12N 5/10, C12N 1/15, C12N 1/19, C12N 1/20, C12N 1/21, C12N 15/61, C12N 15/63, C12N 9/90, C12N 11/02

(54) **NOVEL KETOSE 3-EPIMERASE**

(30) Priority: 21.11.2018 JP 2018218287
(71) Applicant: National University Corporation Kagawa University, Takamatsu-shi, Kagawa 760-8521 (JP)
(72) Inventor: AKIMITSU, Kazuya, Takamatsu-shi, Kagawa 760-8521 (JP); IZUMORI, Ken, Takamatsu-shi, Kagawa 760-8521 (JP); YOSHIHARA, Akihide, Takamatsu-shi, Kagawa 760-8521 (JP); KATO, Shiro, Takamatsu-shi, Kagawa 760-8521 (JP); MOCHIZUKI, Susumu, Takamatsu-shi, Kagawa 760-8521 (JP); YOSHIDA, Hiromi, Takamatsu-shi, Kagawa 760-8521 (JP); KAMITORI, Shigehiro, Takamatsu-shi, Kagawa 760-8521 (JP)
(74) Representative: TBK
(86) International application number: PCT/JP2019/045672
(87) International publication number: WO 2020/105709

(57) **Abstract**

To provide a ketose 3-epimerase that can be used in the food industry and is highly safe, highly active, and heat resistant, a microorganism that produces this enzyme, and a method of producing a ketose. The present invention provides a ketose 3-epimerase that can be obtained from a microorganism belonging to the genus Arthrobacter, has a molecular mass of about 36 kDa as measured by SDS-PAGE, and has the following substrate specificities (A) and (B):
(A) having an activity of epimerizing position 3 of a D- or L-ketose and preparing a D- or L-ketose corresponding thereto, and
(B) having the highest epimerization activity on position 3 of D-allulose, among D- or L-ketohexoses.

## Description

### TECHNICAL FIELD

The present invention relates to a novel ketose 3-epimerase and a method of producing the same, a microorganism producing the same, a DNA encoding the enzyme and a recombinant vector and a transformed host cell containing the DNA, a ketose 3-epimerase mutant, and a method of producing a ketose with the ketose 3-epimerase or the mutant.

### BACKGROUND ART

Rare sugar is a generic name of monosaccharides that do not occur or occur in trace amounts in the natural world. Of hexoses, allose, altrose, gulose, idose, and talose are included in rare sugars as aldoses and allulose (psicose), sorbose, and tagatose are included as ketoses. L-forms, that is, L-fructose and L-glucose are also included in rare sugars.

Researches of a production technology of rare sugars and researches on their physiological action and chemical properties are ongoing and specific physiological actions have been elucidated one after another to date. For industrialization of them as drugs, agricultural chemicals, functional foods, and the like, however, development of an enzyme capable of specifically reacting with rare sugars and enabling mass production of them is indispensable.

D-allulose is also called "D-psicose". It is an epimer of D-fructose and has a sweetness intensity of about 70% of that of sugar. It is similar to D-fructose in sweetness quality. However, it has been made clear that different from D-fructose, D-allulose is hardly metabolized during absorption into the body, has almost zero calories, and has a function of reducing abdominal fat by suppressing the activity of a lipid synthetase. It has so far been reported that D-allulose is usable as a low-calorie sweetener (Patent Document 1) and as a sweetener effective for body weight reduction (Non-Patent Documents 1 and 2). Patent Document 2 describes the use of D-allulose for health foods, foods and beverages for diabetic patients, foods and beverages for slimming, and the like, paying attention to the blood glucose increase suppressing action of D-allulose.

D-tagatose has a sweetness intensity of about 90% of that of sugar, has an energy of 2 kcal/g, and is used as a low-calorie sweetener. It has also been found to have a HDL-cholesterol increasing effect or a body fat accumulation suppressing effect (Patent Document 3) and in addition, have a postrandial blood glucose elevation suppressing action similar to D-allulose.

For the production of these rare sugars, a method using an isomerase is currently employed because of the discovery that it has a useful enzymatic reaction. Ketose 3-epimerase, one of isomerases, can have a plurality of ketoses as a substrate and for example, an enzyme having the highest epimerization activity on position 3 of D-tagatose may be called "D-tagatose 3-epimerase" by inserting the name of a ketose having the highest epimerization activity at position 3 among ketoses serving as a substrate. A technology of producing rare sugar D-allulose from D-fructose has been established by making use of this D-tagatose 3-epimerase (DTE).

For example, Non-Patent Document 3 discloses D-ketose 3-epimerase derived from a Pseudomonas cichorri ST-24 strain and according to it, D-allulose can be produced from D-fructose by making use of this enzyme. However, as can be seen from the fact that it is called by another name "D-tagatose 3-epimerase", its epimerization activity on position 3 of D-tagatose is the highest and an action on D-fructose is relatively weak.

Further, Patent Document 4 has reported a method of preparing D-psicose (D-allulose) by using D-psicose 3-epimerase derived from Agrobacterium tumefaciens and Patent Document 5 has reported a method of producing D-allulose by using ketose 3-epimerase derived from Arthrobacter globiformis, but the activity of these ketose 3-epimerases is not high enough to enable the mass production of D-allulose or D-tagatose on an industrial scale.

### Prior Art

### Patent Documents

Patent Document 1: Japanese Patent No. 4473980
Patent Document 2: Japanese Patent No. 5421512
Patent Document 3: Japanese Patent No. 5749880
Patent Document 4: Japanese Patent No. 4648975
Patent Document 5: Japanese Patent No. 5997693

### Non-Patent Documents

Non-Patent Document 1: Asia Pac. J. Clin. Nutr. (2001) Vol. 10, p. 233-237
Non-Patent Document 2: Asia Pac. J. Clin. Nutr. (2004) Vol. 13, S127
Non-Patent Document 3: Biosci. Biotech. Biochem. (1993) Vol. 57, p. 1037-1039

### SUMMARY

### Technical Problem

An object of the present invention is to provide a novel ketose 3-epimerase which is derived from a microorganism permitted to use for the production of foods and recognized as non-toxic, has high activity and heat resistance, and is capable of catalyzing isomerization from D-fructose to D-allulose at high activity; to provide a method of producing the enzyme; to provide a microorganism which produces the enzyme; to provide a DNA encoding the enzyme and a recombinant vector and a transformed host cell containing it; to provide an immobilized enzyme having the enzyme immobilized thereon and a ketose 3-epimerase mutant protein having improved heat resistance; to provide an immobilized protein having the mutant protein immobilized thereon; and to provide a method of producing a ketose by using the enzyme, immobilized enzyme, mutant protein, or immobilized protein.

### Solution to Problem

Paying attention to the kind of bacteria approved for use as foods not only in Japan but also in Europe and the United States, already on the list, and regarded to be almost non-toxic, the present inventors sampled the soil from various regions, isolated a microorganism therefrom, and continued searching for a microorganism having ketose 3-epimerase activity.

As a result, they have found a microorganism belonging to the genus Arthrobacter and producing a novel ketose 3-epimerase from many isolated strains. An Arthrobacter histidinolovorans Y586-1 strain which is a microorganism belonging to the genus Arthrobacter produces a novel ketose 3-epimerase having a high activity and high heat resistance.

This novel ketose 3-epimerase derived from a microorganism has an activity of acting on position 3 of a D or L-ketose and catalyzes epimerization into a corresponding D- or L-ketose. It has the highest epimerization activity on position 3 of D-allulose among D- and L-ketohexoses and is suited for the production of D-allulose from D-fructose.

Described specifically, the present invention relates to a ketose 3-epimerase described in the following (1) to (5).
(1) A ketose 3-epimerase derived from a microorganism belonging to the genus Arthrobacter, having a molecular mass of 36 kDa as measured by SDS-PAGE, and having the following substrate specificities (A) and (B):
   (A) having an activity of epimerizing position 3 of a D- or L-ketose and preparing a D- or L-ketose corresponding thereto, and
   (B) having the highest epimerization activity on position 3 of D-allulose among D- or L-ketohexoses.
(2) The ketose 3-epimerase described above in (1), having the following physicochemical properties (C) and (D):
   (C) having an optimum reaction pH or from 6 to 8, and
   (D) having an optimum reaction temperature of 60°C.

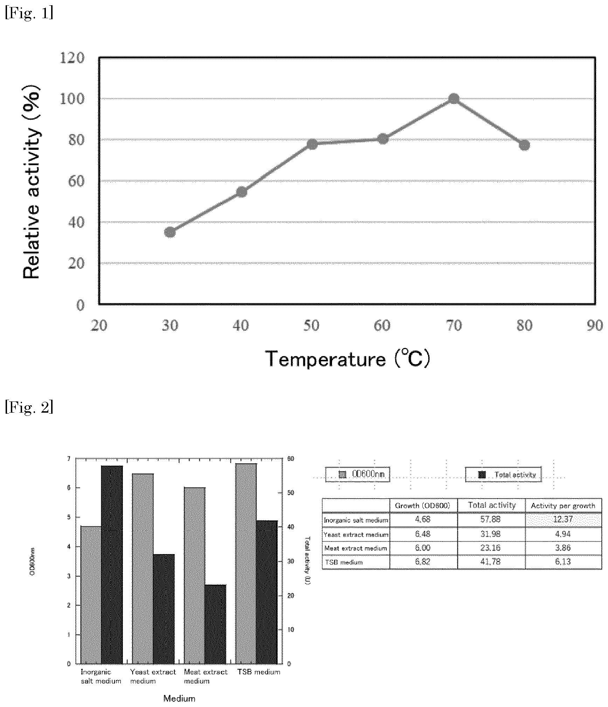

The above-described optimum reaction temperature 60°C is that of a purified enzyme. In Examples, two experiments were performed using a purified enzyme and a crude enzyme, respectively. The crude enzyme showed an optimum reaction temperature of 70°C (refer to Fig. 1). The crude enzyme showed 70°C and the purified enzyme showed 60°C. Such an example where an enzyme in crude form has increased heat resistance due to an influence of a protein or the like therearound is an often found phenomenon. The paragraph 0047 describes that with regard to a recombinant mutant, a large number of mutants having higher heat resistance than a wild type ketose 3-epimerase can be obtained, which can be understood from that they can be obtained from a crude enzyme.
(3) The ketose 3-epimerase described above in (1) or (2), wherein the substrate specificity is in the order of D-allulose, D-tagatose, L-tagatose, D-fructose, and L-allulose.
(4) The ketose 3-epimerase described above in any of (1) to (3), wherein the microorganism belonging to the genus Arthrobacter is Arthrobacter histidinolovorans.
(5) The ketose 3-epimerase described above in any of (1) to (4), wherein the microorganism belonging to the genus Arthrobacter is an Arthrobacter histidinolovorans Y586-1 strain deposited under an accession number NITE BP-02813 at the Patent Microorganisms Depositary Center.

The present invention also relates to a protein described in the following (6) to (8), a DNA, recombinant vector, or transformed host cell described in the following (9) to (13), or a microorganism described in the following (14).
(6) A protein described in the following (a) or (b):
   (a) a protein having an amino acid sequence represented by SEQ ID NO: 1, or
   (b) a protein having an amino acid sequence having 70% more identity to the amino acid sequence represented by SEQ ID NO: 1, having the following ketose 3-epimerase activities (A) and (B), and having a ketose 3-epimerase activity ratio (T70/T50) of 1.55 or more at a reaction temperature of 70°C to 50°C:
      (A) having an activity of epimerizing position 3 of a D- or L-ketose and preparing a D- or L-ketose corresponding thereto, and
      (B) having the highest epimerization activity on position 3 of D-allulose among D- or L-ketohexoses.
(7) A protein described in the following (a) or (b):
   (a) a protein having an amino acid sequence represented by SEQ ID NO: 1, or
   (b) a protein having an amino acid sequence having 70% more identity to the amino acid sequence represented by SEQ ID NO: 1, having the following ketose 3-epimerase activities (A) and (B), and having a residual activity of 31% or more after incubation at 60°C for one hour:
      (A) having an activity of epimerizing position 3 of a D- or L-ketose and preparing a D- or L-ketose corresponding thereto, and
      (B) having the highest epimerization activity on position 3 of D-allulose among D- or L-ketohexoses.
(8) A protein composed of an amino acid-substituted mutant of a protein having an amino acid sequence represented by SEQ ID NO: 1, wherein the mutant has amino acid substitution at at least one site selected from H56, T59, A60, P77, L94, L97, D101, A109, A128, V129, V132, S144, S167, P172, E199, A200, K202, and S218 of the amino acid sequence represented by SEQ ID NO: 1, has the following ketose 3-epimerase activities (A) and (B), and has a higher ketose 3-epimerase activity ratio (T70/T50) at a reaction temperature of 70°C to 50°C or having a higher residual activity after incubation at 60°C for one hour than the protein having an amino acid sequence represented by SEQ ID NO: 1:
   (A) having an activity of epimerizing position 3 of a D- or L-ketose and preparing a D- or L-ketose corresponding thereto, and
   (B) having the highest epimerization activity on position 3 of D-allulose among D- or L-ketohexoses.
(9) A DNA encoding the protein described above in any of (6) to (8).
(10) A DNA having a base sequence represented by SEQ ID NO: 2 or a complementary sequence thereof.
(11) The DNA described above in (9), characterized in that the DNA is any of the following ones (a) to (c):
   (a) a DNA having a sequence containing the whole or a part of a base sequence represented by SEQ ID NO: 2 or a complementary sequence thereof,
   (b) a DNA that hybridizes with a complementary strand of a DNA having a base sequence represented by SEQ ID NO: 2 under stringent conditions, and
   (c) a DNA having 70% or more sequence identity to a DNA having a base sequence represented by SEQ ID NO: 2.
(12) A recombinant vector containing the DNA described above in any of (9) to (11).
(13) A transformed host cell obtained by transformation by the recombinant vector described in (12).
(14) An Arthrobacter histidinolovorans Y586-1 strain that produces the ketose 3-epimerase described above in any of (1) to (3) and is deposited under an accession number NITE BP-02813 at the Patent Microorganisms Depositary Center.

Further, the present invention relates to an immobilized enzyme described in the following (15) to (17) and an immobilized protein described in the following (18) and (19).
(15) An immobilized enzyme obtained by immobilizing the ketose 3-epimerase described above in any of (1) to (5) on a carrier.
(16) An immobilized enzyme characterized in that a ketose 3-epimerase crude enzyme present in a disrupted cell of a microorganism belonging to the genus Arthrobacter that produces the ketose 3-epimerase described above in any of (1) to (5) has been immobilized on a carrier.
(17) The immobilized enzyme described above in (15) or (16), wherein the carrier is an ion exchange resin, an organic polymer carrier, and/or an inorganic carrier.
(18) An immobilized protein, characterized in that the protein described above in any of (6) to (8) has been immobilized on a carrier.
(19) The immobilized protein described above in (18), wherein the carrier is an ion exchange resin, an organic polymer carrier, and/or an inorganic carrier.

Further, the present invention relates to a method of producing a ketose 3-epimerase described in the following (19) and (20), a method of producing a protein having a ketose 3-epimerase activity described in the following (21), and a method of producing a ketose described in the following (22).
(19) A method of producing a ketose 3-epimerase, including culturing, in a medium, a microorganism belonging to the genus Arthrobacter that produces the ketose 3-epimerase described above in any of (1) to (5), accumulating the ketose 3-epimerase in a microorganism cell, and collecting the resulting ketose 3-epimerase.
(20) The method of producing a ketose 3-epimerase described above in (19), wherein the medium is a D-allulose-added inorganic salt medium.
(21) A method of producing a protein having a ketose 3-epimerase activity, including culturing the transformed host cell described above in (13) in a medium and collecting a protein having a ketose 3-epimerase activity from the cultured product.
(22) A method of producing a ketose, including allowing the ketose 3-epimerase described above in any of (1) to (5), the protein described above in any of (6) to (8), the immobilized enzyme described above in any of (15) to (17), or the immobilized protein described above in either of (18) or (19) to act on a solution containing at least one ketose selected from D- or L-ketoses to epimerize position 3 of the ketose and produce a ketose corresponding thereto and collecting the resulting ketose.

### Advantageous Effects of Invention

The ketose 3-epimerase of the present invention is characterized by having particularly higher activity than the conventional microorganism-derived ketose 3-epimerase. For example, compared with the ketose 3-epimerase derived from Arthrobacter globiformis described in Patent Document 5, the enzyme produced by the present invention has an about 2 times higher activity per culture liquid so that use of the enzyme enables the efficient mass production of D-allulose.

The enzyme has an optimum temperature as high as 70°C and even at 80°C, it has a relative activity of 59.4 supposing that the activity at the optimum temperature of 70°C is 100. It is almost about 60%, showing high heat resistance. In addition, the enzyme has a residual activity of 49.4% after incubation at 60°C for one hour and has heat tolerance so that it enables production of a larger amount of a product in the reaction at the optimum temperature of 70°C. This enzyme having such excellent heat resistance is characterized in that when an amino acid substituted mutant is prepared therefrom, the resulting mutant can have more enhanced heat resistance.

One of the advantages of using the present enzyme derived from a microorganism belonging to the genus Arthrobacter in the food industry is safety of the microorganism.

### BRIEF DESCRIPTION OF DRAWINGS

Fig. 1 is a drawing showing the optimum temperature of the present crude enzyme.
Fig. 2 is a drawing showing the influence of a medium on an enzyme production amount.
Fig. 3 shows the results of SDS-PAGE for confirming the molecular mass of the present enzyme, wherein the unit of the left number is kDa.
Fig. 4 is a drawing showing the optimum pH of the present purified enzyme and a control enzyme.
Fig. 5 is a drawing showing the optimum temperature of the present purified enzyme.
Fig. 6 is a drawing showing the temperature stability of the present purified enzyme and the control enzyme after incubation at various temperatures for 10 minutes.
Fig. 7 is a drawing showing the substrate specificity of the present purified enzyme.
Fig. 8 shows the respective relative activities of the present enzyme immobilized on carriers HPA25L, WA30, IRA904CL, FRA54, FRA95, and XAD7HP.
Fig. 9 shows the respective relative activities of the enzyme immobilized on the carriers at from 30°C to 80°C in the presence or absence of Mg²⁺.
Fig. 10 shows the MALDI-TOF-MS analysis results of a fragment obtained by subjecting the SDS-PAGE 36 kDa band of Fig. 3 to reduction treatment and alkylation treatment and then digesting it with trypsin. In other words, shown is the peptide peak of a trypsin-digested 36 kDa protein obtained by MALDI-TOF-MS analysis.
Fig. 11 shows the results of SDS-PAGE of a recombinant enzyme expressed in Escherichia coli. Shown in contrast is the SDS-PAGE of the recombinant enzyme and the purified enzyme derived from the Y586-1 strain. The recombinant enzyme has, at the C terminal thereof, an additional sequence RSHHHHHH and is about 1 kDa greater than the Y586-1 strain-derived purified enzyme. In the drawing, the unit of the left number is kDa.
Fig. 12 is a drawing showing the three-dimensional structure of the present enzyme (purified recombinant enzyme) by X-ray crystallography.

### DESCRIPTION OF EMBODIMENTS

The present invention relates to a ketose 3-epimerase which can be isolated from a microorganism belonging to the genus Arthrobacter. The enzyme can be produced much per culture liquid and exhibits a characteristic property in heat stability.

Microorganisms belonging to the genus Arthrobacter are safe ones listed in the Japanese food additive list as microorganisms serving as an origin of an enzyme such as α-amylase, isomaltdextranase, invertase, urease, glucanase, α-glucosyltransferase, and fructosyltransferase, and also trehalose- or vitamin K (menaquinone) -producing bacteria.

The term "ketose" is a chemical term used for classification of a saccharides by its structure and it is a generic name of monosaccharides having, in the chain structure thereof, one keto group (ketonic carbonyl group). The molecular formula of it is CₙH₂ₙOₙ (n≥3). Representative ketoses include fructose but they also include pentose and the like.

In the present invention, the term "ketose" means ketose in the broad sense and it includes pentose and the like. The term "ketohexose" means a hexose having a ketose structure, that is, ketohexose. More specifically, it means fructose, allulose, tagatose, and sorbose and the term "D-ketose" or "L-ketose" means "D-form" or "L-form" of them.

The ketose 3-epimerase of the present invention therefore has the activity of epimerizing position 3 of a D- or L-ketose to prepare a D- or L-ketose corresponding thereto. More specifically, it can catalyze the interconversion between D- or L-fructose and D- or L-allulose and interconversion between D- or L-tagatose and D- or L-sorbose. Further, it can be allowed to act on all other ketoses.

The ketose 3-epimerase of the present invention can be prepared by culturing a microorganism belonging to the genus Arthrobacter and having ketose 3-epimerase production ability and isolating the resulting ketose 3-epimerase from the cells grown in the culture liquid. As the microorganism belonging to the genus Arthrobacter, for example, an Arthrobacter histidinolovorans Y586-1 strain and mutant strains thereof are usable advantageously. In filing the present application, the Y586-1 strain was internationally deposited on November 7, 2018 at the National Institute of Technology and Evaluation, Patent Microorganisms Depositary Center located at 2-5-8 Kazusa Kamatari, Kisarazu, Chiba, Japan, and was deposited under the accession number NITE BP-02813.

With respect to the Y586-1 strain, the homology search of the base sequence of a 16SrRNA gene to known strains was performed by BLAST search (Japan DNA Data Bank) and from the name of the strain having 98% or more homology to the base sequence, it was initially identified as Arthrobacter protophormiae. After that, Japan Food Analysis Center identified it as Arthrobacter histidinolovorans so that the sequence search of the complete genome of the Y586-1 strain was performed to confirm that it was Arthrobacter histidinolovorans.

The renaming of the strain with an accession number NITE BP-02813 was notified to the National Institute of Technology and Evaluation, Patent Microorganisms Depositary Center and was approved on October 4, 2019.

The crude enzyme solution of the ketose 3-epimerase of the present invention is prepared by performing the aeration culture of ketose 3-epimerase producing bacteria belonging to the genus Arthrobacter in a D-allulose-added inorganic salt medium; collecting the cells from the culture liquid by centrifugal separation; washing the cells thus collected with a 10 mM tris-HCl buffer (pH 7.5); suspending them in 10 ml of a 10 mM tris-HCl buffer (pH 7.5); carrying out an enzymatic treatment with a lysozyme added as a lytic enzyme to disrupt the cells or disrupting the cells with a ultrasonic homogenizer while cooling the cell suspension in ice water; and centrifuging the disrupted product to obtain a centrifugation supernatant.

The activity of the ketose 3-epimerase in the crude enzyme solution before purification can be found by measuring a preparation amount of D-allulose with D-fructose as a substrate.

The enzyme activity of a reverse reaction, that is, epimerization from D-allulose to D-fructose is also measured under similar conditions. The ketose exchange reaction is usually performed under the following conditions. The substrate concentration is from 1 to 60% (w/v), desirably from about 5 to 50% (w/v), the reaction temperature is from 10 to 70°C, desirably from about 30 to 60°C, and the reaction pH is from 5 to 10, desirably from about 7 to 10. Although the reaction time can be selected as needed, the reaction time in a batch reaction is usually selected from a range of from 5 to 50 hours.

The crude enzyme solution is fractionated by changing the concentration of polyethylene glycol (PEG), subjecting the supernatant having PEG and the ketose 3-epimerase therein to ion exchange resin chromatography to allow adsorption of the intended enzyme, rinsing away the PEG, and then, eluting the adsorbed enzyme. The enzyme elution solution is then purified by ion exchange resin chromatography and hydrophobic chromatography successively and thus, a purified enzyme can be isolated.

To confirm the purification of the enzyme, SDS-PAGE (gel concentration: 12.5%) is performed to confirm that a single band can be obtained and also confirm its molecular mass.

The ketose 3-epimerase of the present invention thus purified is a metal enzyme which exhibits a molecular mass of about 36 kDa in SDS-PAGE and whose activation is adjusted by a metal ion. The reaction with a substrate is performed in the presence of a metal ion selected from the group consisting of manganese, magnesium, iron, cobalt, and aluminum at a concentration of from 0.5 to 5 mM.

The ketose 3-epimerase of the present invention has a predetermined amino acid sequence. However, the gene of the present enzyme could not be cloned using a PCR amplification method or existing protein data base so that the complete genome sequence of the Y586-1 strain was determined and data base of a protein group encoding all the ORFs in the resulting genome sequence was constructed. A fragment obtained by treating the purified enzyme with trypsin was analyzed by MALDI-TOF-MS and it matched the amino acid of the fragment thus obtained, making it possible to finally determine the amino acid sequence of the ketose 3-epimerase of the present invention.

Examples of it include proteins having an amino acid sequence represented by SEQ ID NO: 1 and proteins having an amino acid sequence homologous thereto and maintaining an equivalent ketose 3-epimerase activity.

The term "having an amino acid sequence homologous thereto" means, for example, having an amino acid sequence 70% or more, preferably 75% or more, more preferably 76, 77, 78, 79, or 80% or more, still more preferably 85% or more, still more preferably 86, 87, 88, 89, 90, 91, 92, 93, or 94% or more, still more preferably 95%, 96%, 97%, or 98% or more identical to the amino acid sequence of SEQ ID NO: 1.

The identity (%) of two amino acid sequences or two nucleic acid sequences (base sequences) can be determined, for example, by the following procedure. First, two sequences are arranged for the optimal comparison. At this time, for example, a gap may be introduced into the first sequence to optimize the alignment with the second sequence. When a molecule (amino acid residue or nucleotide) at a specific position in the first sequence and a molecule at a position corresponding thereto in the second sequence are the same, the molecules at the position are said to be identical. The identity of the two sequences is a function of the number of the same positions common to these two sequences (that is, identity (%) = number of same positions/total number of positions × 100) and the number and size of the gap needed for the optimization of the alignment is preferably taken into consideration.

In addition, the comparison of the two sequences and determination of the identity can be achieved using a mathematical algorism. Specific examples of the mathematical algorism usable for sequence comparison include, but not limited to, the algorism described in Karlin and Altschul (1990) Proc. Natl. Acad. Sci. USA 87: 2264-68 and altered in Karlin and Altschul (1993) Proc. Natl. Acad. Sci. USA 90: 5873-77. Such an algorism has been incorporated in the NBLAST program and XBLAST program (version 2.0) described in Altschul et al., (1990) J. Mol. Biol. 215: 403-10. A nucleotide sequence equivalent to the nucleic acid molecule of the present invention may be obtained, for example, by performing BLAST nucleotide search with score=100 and wordlength=12 by using the NBLAST program.

The DNA of the present invention is a gene encoding the ketose 3-epimerase of the present invention and has a predetermined base sequence. Examples of it include a DNA sequence encoding the amino acid sequence represented by SEQ ID NO: 1, a base sequence represented by SEQ ID NO: 2, and a DNA sequence having a base sequence homologous to the base sequence represented by SEQ ID NO: 2 and encoding a protein maintaining a ketose 3-epimerase activity equivalent to that of the protein of SEQ ID NO: 1. A DNA having the base sequence represented by SEQ ID NO: 2 or a complementary sequence thereto is a novel compound.

The DNA of the present invention can be isolated by using known techniques, for example, a hybridization technique, a PCR technique and the like in combination.

The DNA of the present invention includes a DNA having the base sequence represented by SEQ ID NO: 2, a DNA which hybridizes with the above DNA and a complementary strand of the above DNA under stringent conditions, and a DNA having 70% or more sequence identity to the above DNA or the complementary chain of the above DNA.

The term "stringent conditions" as used herein means hybridization conditions readily determined by those skilled in the art and in general, they are empirical experimental conditions depending on a probe length, washing temperature, and salt concentration. Usually, with an increase in the probe length, the temperature for appropriate annealing increases. With a decrease in the probe length, the temperature decreases. Formation of a hybrid between nucleic acids depends on the reannealing ability of the complementary chain under an environment slightly lower than the melting point thereof. More specifically, the stringent conditions which enable isolation of the DNA are not particularly limited insofar as they are selected by those skilled in the art. For example, in the washing stage, preferred are conditions under which a temperature is from 42°C to 70°C, preferably from 42°C to 65°C and a sodium concentration of from 25 mM to 450 mM, preferably from 25 mM to 500 mM. Examples of such conditions include washing with 5X SSC (83 mM NaCl, 83 mM sodium citrate) in 0.1% SDS at 42°C. A DNA having high homology can be obtained by making the conditions more stringent, for example, by increasing the temperature. The term "washing under stringent conditions" means washing three times each for 15 minutes at 45°C (very low stringency), 50°C (low stringency), 55°C (moderately high stringency), 60°C (medium high stringency), 65°C (high stringency), or 70°C (very high stringency) while using 5X SSC and 0.2% SDS solution finally.

The term "base sequence homologous to" means "DNA having 70% or more sequence identity to" and % is not limited insofar as it is a DNA having 70% or more sequence identity to the base sequence of SEQ ID NO: 2. For example, it may be 70% or more, preferably 75% or more, more preferably 76, 77, 78, 79 or 80% or more, still more preferably 85% or more, still more preferably 86,87, 88, 89, 90, 91, 92, 93, or 94% or more, still more preferably 95%, 96%, 97%, or 98% or more identity to the base sequence.

The DNA of the present invention can be inserted into an autonomously replicable and appropriate vector to obtain a recombinant vector. The recombinant vector is composed of a DNA and an autonomously replicable vector so that it can be prepared relatively easily by the conventional recombination DNA technology if a DNA can be obtained. An appropriate vector is selected depending on the using purpose such as cloning or protein expression, or depending on host cells. Examples of such a vector include plasmid vectors such as pBR322, pUC18, pUB110, pTZ4, pC194, pHV14, TRp7, YEp7, and pBS7 and phage vectors such as λgt·λC, λgt·λB, ρ11, ϕ1, and ϕ105.

The recombinant vector thus obtained can be introduced into appropriate host cells including Escherichia coli, Bacillus subtilis, Actinomycetes, and yeast. It is introduced by a known method such as calcium phosphate coprecipitation method, electroporation method, lipofection method, and microinjection method. A colony hybridization method or the like is applied to obtain transformed host cells.

A method of producing the protein of the present invention having a ketose 3-epimerase activity is not particularly limited and a known method can be used. Described specifically, the protein having a ketose 3-epimerase activity can be collected and thereby produced from a cultured product obtained by culturing, in a nutrient medium, a microorganism having a production ability of the ketose 3-epimerase of the present invention or host cells transformed with a DNA encoding the protein of the present invention having ketose 3-epimerase ability or an amino acid-substituted mutant of the protein which will be described later. For the culturing, a known method can be used and for example, either liquid culture or solid culture can be used.

After the cells are cultured in such a manner, the protein of the present invention is purified · collected. The protein purification · collection method can be selected freely from known methods. For example, the protein can be collected from the culture liquid by removing an insoluble matter from the culture supernatant, for example, by filtration, centrifugation treatment, or the like and then performing separation and purification by using, in combination, enrichment through a filtration membrane, salting-out such as ammonium sulfate precipitation, dialysis, and various chromatographies using an ion exchange resin or the like.
On the other hand, for the collection of the protein from the cells, for example, the cells are disrupted by treatment with a lytic enzyme, ultrasonic treatment, or the like, followed by similar separation and purification.

Further, the ketose 3-epimerase of the present invention can be used in the form of an immobilized enzyme similar to other epimerases or isomerases. An immobilized enzyme with high activity can be obtained by various immobilization methods. Using an immobilized enzyme enables a continuous and mass epimerization reaction. An immobilized enzyme can be obtained by making use of a known immobilization means, for example, carrier binding method, crosslinking method, gel encapsulation method, and microcapsulation method. The carrier may be any carrier. In the carrier binding method, the enzyme can be bound to a carrier (for example, derivatives of a polysaccharide such as cellulose, dextran, and agarose, polyacrylamide gel, polystyrene resin, porous glass, and metal oxide) by physical adsorption, ionic binding, covalent binding, or the like. In the crosslinking method, a reagent having two or more functional groups is used to crosslink enzymes with each other to immobilize them. As the crosslinking reagent, glutaraldehyde for forming a Schiff base, an isocyanic acid derivative for peptide binding, N,N'-ethylenemaleimide, bisdiazobenzine for diazo coupling, N,N'-polymethylene bisiodoacetamide for alkylation, and the like are usable. In the gel encapsulation method, a lattice type which incorporates the enzyme in fine lattices of a polymer gel and a microcapsule type which covers the enzyme with a semitransparent polymer film are used. The lattice type method can use synthetic polymer substances such as polyacrylamide gel, polyvinyl alcohol, and photocuring resins and natural polymer substances such as starch, conjaku powder, gelatin, alginic acid, and carrageenan. The microcapsule type method can use hexamethylenediamine, sebacoyl chloride, polystyrene, lecithin, and the like. The above five types of enzymes may be immobilized on a carrier, respectively, or a combination of 2, 3, 4, or 5 enzymes selected from them may be immobilized.

In one aspect of the method of immobilizing the ketose 3-epimerase of the present invention, immobilization is performed using a crude enzyme solution. The ketose 3-epimerase can be immobilized by adding a ketose 3-epimerase-containing crude enzyme solution obtained by the ultrasonic treatment of the cell suspension to an ion exchange resin or the like and binding the former to the latter at low temperatures.

For taking out the ketose 3-epimerase from the cells, the cell wall should be disrupted. As described above, there are disruption methods by ultrasonic treatment and by treatment with an enzyme such as lysozyme. It has been found that a crude enzyme solution obtained by the ultrasonic treatment is rich in a ketose 3-epimerase having an activity.

As the carrier on which the enzyme from the crude enzyme solution obtained as described above is immobilized, any known immobilization carrier can be used. Various ion exchange resins, synthetic adsorbents, and the like are convenient so that they are frequently used. An encapsulation method with sodium alginate can also be used.

The immobilization carrier to be used for ketose 3-epimerase immobilization is not particularly limited insofar as the enzyme can be immobilized on it. Examples include organic polymer carriers such as ion exchange resins, polyvinyl alcohol, polypropylene, acryl, chitosan, hydrophobic adsorption resins, chelate resins, and synthetic adsorption resins; and inorganic carriers such as celite, diatomaceous earth, kaolinite, silica gel, molecular sieves, porous glass, activated carbon, and ceramics. When a microorganism is used as an immobilized enzyme, a quaternized pyridine compound (for example, "DAC", trade name; product of Denka) and an alginate can be used as an immobilization carrier. These immobilization carriers may be used either singly or in combination of two or more. Of these immobilization carriers, ion exchange resins are preferred from the standpoint of maintaining the activity of a ketose 3-epimerase by immobilization, a low cost, less pressure loss, and handling ease. For example, as a basic anion exchange resin, either a strongly basic anion exchange resin or a weakly basic anion exchange resin can be used. Examples of the strongly basic anion exchange resin include HPA25L (product of Mitsubishi Chemical) and IRA904CL (product of Organo), while those of the weakly basic anion exchange resin include WA30 (product of Mitsubishi Chemical) and FPA54 and FPA95 (products of Organo). Examples of the synthetic adsorbent include XAD7HP (product of Organo).

When an immobilized enzyme is produced using the weakly basic anion exchange resin as a carrier, the immobilized ketose 3-epimerase can be eluted easily after disappearance of its isomerizability so that the immobilized carrier can be recovered very conveniently and a production cost can therefore be reduced.

As in the description about the immobilized enzyme obtained by immobilizing, on a carrier, the ketose 3-epimerase crude enzyme present in disrupted cells of a ketose 3-epimerase-producing microorganism belonging to the genus Arthrobacter, an immobilized ketose 3-epimerase having a purified product or crude purified product of a ketose 3-epimerase immobilized thereon or a ketose 3-epimerase-producing microorganism immobilized thereon may be used in the present invention. Further, it is needless to say that an immobilized protein of a recombinant enzyme expressed in Escherichia coli, like the crude enzyme or purified enzyme, immobilized thereon may be used.

By epimerizing position 3 of a ketose which will be a substrate with the ketose 3-epimerase of the present invention, it can be converted into a corresponding ketose. Thus, the corresponding ketose can be produced. The ketose 3-epimerase of the present invention has a high epimerization activity on position 3 of D-allulose or D-fructose so that a large amount of D-allulose which is a rare sugar as a product can be produced efficiently from D-fructose which will be a substrate.

An enzyme having higher heat resistance than an amino acid substitution-free wild type enzyme can be obtained by introducing mutation into the gene of the ketose 3-epimerase of the present invention and substituting the corresponding amino acid residue with another amino acid residue by a site-directed mutagenesis operation to prepare various amino acid-substituted mutants. The heat resistance is evaluated by two indices, that is, a ketose 3-epimerase activity ratio (T70/T50) at the reaction temperatures of 70°C to 50°C and a residual activity after incubation at 60°C for one hour.

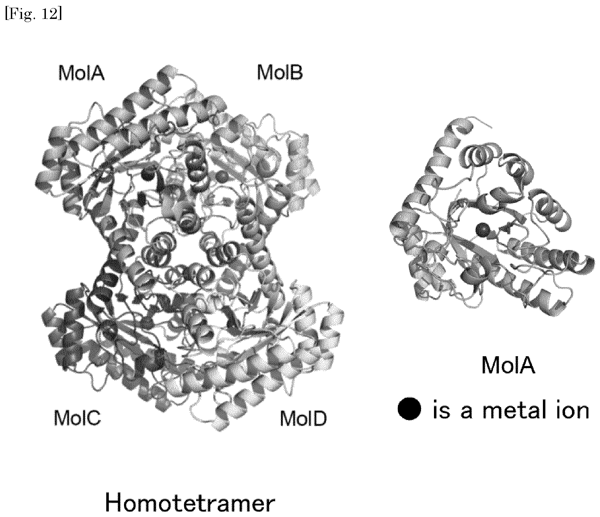

The three-dimensional structure analysis has suggested that the present enzyme is a homotetramer as shown in Fig. 12 and it is presumed that the active site residues of the present enzyme are amino acids at position 6, position 63, position 110, position 152, position 179, position 182, position 205, position 211, and position 240 and residues at a presumed metal ion binding site are amino acids at position 146, position 177, position 205, and position 240. Amino acid substitution in the vicinity of these amino acids is therefore not performed and a mutant is prepared by amino acid substitution at the other sites.

Fig. 12 is a drawing showing the three-dimensional structure of the present enzyme (purified recombinant enzyme) as a result of X-ray crystallography. Crystallization was performed with a purified recombinant enzyme and the structure of the ketose 3-epimerase derived from the Y586-1 strain was determined by X-ray crystal analysis. The present enzyme is a homotetramer (MolA, MolA, MolA, and MolA) in which four subunits have been assembled. The subunit structures , each having a molecular mass of about 36 kDa confirmed by SDS-PAGE, show a β/α barrel structure and have a metal ion bound to the center active site of the structure.

The ketose 3-epimerase of the present invention is a mutant having an amino acid sequence identity on only a 70% level, but surprisingly, it maintains an enzyme activity and at the same time has a ketose 3-epimerase activity ratio (T70/T50) at a reaction temperature of 70°C to 50°C and a residual activity after incubation at 60°C for one hour, each higher than those of a wild type enzyme having SEQ ID NO: 1.

In addition, according to the presumption that sites different in amino acid residue are less involved in the ketose 3-epimerase activity as a result of comparison between the amino acid sequence of the ketose 3-epimerase represented by SEQ ID NO: 1 and the amino acid sequence of the previously reported ketose 3-epimerase (Patent Document 5) derived from Arthrobacter globiformis, amino acids at the different sites in SEQ ID NO: 1 are changed by base substitution by site-directed mutagenesis to form various ketose 3-epimerase mutants and an amino acid-substituted mutant having higher heat resistance than the wild type is selected from them.

The site-directed mutagenesis method can be performed by an arbitrary method, for example, the inverse PCR method or the annealing method (edited by Muramatsu et al., "Revised 4th Edition New Genetic Engineering Handbook", Yodosha, p. 82-88). If necessary, various commercially-available site-directed mutagenesis kits such as QuickChange II Site-Directed Mutagenesis Kit and QuickChange Multi Site-Directed Mutagenesis Ki, product of Stratagene can also be used.

The site-directed mutagenesis can most commonly be performed using a mutation primer including nucleotide mutation to be introduced. Such a mutation primer may be designed so as to anneal to a region containing a nucleotide sequence encoding an intragenal amino acid residue to be altered and include a base sequence having a nucleotide sequence (codon) encoding an amino acid residue after alteration instead of the nucleotide sequence (codon) encoding an amino acid residue to be altered.

The term "amino acid-substituted mutant" means a mutant having, instead of an amino acid of the original sequence, an amino acid different therefrom as a result of substitution. Although the substitution is not particularly limited and it may be conservative substitution or non-conservative substitution, the substitution in a preferred aspect of the present invention is conservative one. The term "conservative substitution" means substitution between amino acids having the same property (basic, acidic, or neutral) or the same polarity (hydrophilic or hydrophobic) or between aromatic amino acids, or between aliphatic amino acids, for example, substitution from basic to basic, acidic to acidic, or polar to polar.

The conservative substitution is performed, for example, within each of the groups of basic amino acids (Arg, Lys, His), acidic amino acids (Glu, Asp), neutral non-polar amino acids (Gly, Ala, Val, Leu, Ile, Met), aliphatic amino acids (Ala, Val, Leu, Ile, Met), polar amino acids (Gln, Asn, Ser, Thr), and aromatic amino acids (Phe, Trp, Tyr).

On the other hand, the term "non-conservative substitution" means "exchange with an amino acid which is a member outside the above group and for example, folding in a protein in a tertiary structure is prevented by deletion of Cys or substitution with another amino acid. In order to keep a hydrophilicity/hydrophobicity balance or enhance hydrophilicity to facilitate synthesis, an amino acid may be substituted in consideration of a hydropathy index (J. Mol. Biol. (1982) Vol. 157, p. 105-132) of an amino acid which is an index of hydrophobicity/hydrophilicity about an amino acid.

Further, an original amino acid may be substituted by an amino acid having less steric hindrance or an amino acid having a charge may be substituted by an amino acid having no charge.

The substitution may be conservative substitution or non-conservative substitution. Examples include, but not limited to, substitution of Ala by Ser or Thr, substitution of Arg by Gln, His, or Lys, substitution of Asn by Glu, Gln, Lys, His, or Asp, substitution of Asp by Asn, Glu, or Gln, substitution of Cys by Ser or Ala, substitution of Gln by Asn, Glu, Lys, His, Asp, or Arg, substitution of Glu by Asn, Gln, Lys, or Asp, substitution of Gly by Pro, substitution of His by Asn, Lys, Gln, Arg, or Tyr, substitution of Ile by Leu, Met, Val, or Phe, substitution of Leu by Ile, Met, Val, or Phe, substitution of Lys by Asn, Glu, Gln, His, or Arg, substitution of Met by Ile, Leu, Val or Phe, substitution of Phe by Trp, Tyr, Met, Ile, or Leu, substitution of Ser by Thr or Ala, substitution of Thr by Ser or Ala, substitution of Trp by Phe or Tyr, substitution of Tyr by His, Phe, or Trp, and substitution of Val by Met, Ile, or Leu. With respect to the substitution of these amino acid residues, the classification of residues which can be substituted is shown only as an example and amino acid residues which can be substituted are not limited to the above classification.

A multiple mutant having 11 substituted amino acids was prepared from a mutant of the present enzyme having one substituted amino acid and a heat resistance test of it was conducted. The following are the results of the test: 30 kinds, among 37 kinds, showed a higher ketose 3-epimerase activity ratio (T70/T50) at a reaction temperature of 70°C to 50°C than a control enzyme (1.54) derived from Arthrobacter globiformis M30 described in Patent Document 5; and not only 37 kinds, among 37 kinds, showed a higher residual activity after incubation at 60°C for 1 hour than the globiformis-derived enzyme (30.8%) but also 36 kinds, among 37 kinds, showed a higher residual activity than the wild type enzyme of the present invention represented by SEQ ID NO: 1.

The ketose 3-epimerase of the present invention is also excellent in providing a number of mutants having higher heat resistance than the wild type ketose 3-epimerase.

### Examples

The present invention will hereinafter be described in detail by experiments.

In the following experiments, a D-allulose 3-epimerase activity for converting D-allulose into D-fructose may be measured and referred to as "ketose 3-epimerase activity" ,and a D-fructose 3-epimerase activity for converting D-fructose into D-allulose may be measured and referred to as "ketose 3-epimerase activity". The present invention is not limited to or by the following Examples.

### <Experiment 1: Origin and identification of strain>

The present inventors measured the activity of ketose 3-epimerase by inoculating a number of bacteria isolated by screening in a D-allulose-added liquid medium, conducting shaking culture, and measuring the preparation amount of D-allulose with D-fructose as a substrate.

In such a manner, a microorganism Y586-1 strain was found as a strain having the highest activity and as a result of system analysis based on the homology to a 16SrRNA gene base sequence, the Y586-1 strain was found to belong to Arthrobacter protophormiae .

### Identification of strain

### (1) Homology to a 16SrRNA gene base sequence

A 16SrRNA gene region was analyzed and the number of bases was identified as 1,469 bp.

### (2) Homology search

The homology search of the base sequence of the 16SrRNA gene of the strain to a known strain was performed by BLAST search (Japan DNA Data Bank). Based on the name and homology value (%) of the strains having 98% or more homology to the base sequence having a base number of 1,469 bp identified above, the microorganism of the Y586-1 strain was identified as Arthrobacter protophormiae. The Y586-1 strain was internationally deposited on November 7, 2018 at the National Institute of Technology and Evaluation, Patent Microorganisms Depositary Center and was deposited as accession number NITE BP-02813. Since afterwards it was judged as Arthrobacter histidinolovorans, renaming of the strain of the accession number NITE BP-02813 was notified to the National Institute of Technology and Evaluation, Patent Microorganisms Depositary Center and was approved on October 4, 2019.

### <Experiment 2: Culturing of Arthrobacter histidinolovorans Y586-1 strain (Accession number: NITE BP-02813>

A 1% (v/v) seed culture liquid of Arthrobacter histidinolovorans Y586-1 strain (Accession number: NITE BP-02813) was aseptically added to an inorganic salt medium having 1.0% D-allulose as a carbon source and ammonium sulfate as a nitrogen source and cultured at 30°C for 24 hours while aerating and stirring. The activity of the ketose 3-epimerase in the resulting culture liquid was about 2.694 units/100 mL culture liquid. The strain will hereinafter be abbreviated and simply called "Y586-1 strain".

A 1% (v/v) seed culture liquid of the Y586-1 strain was aseptically added to each of an inorganic salt liquid medium, yeast extract liquid medium, meat extract liquid medium, and TSB liquid medium, each containing 1.0% D-allulose and cultured at 30°C for 24 hours while aerating and stirring. The growth (OD₆₀₀ₙₘ) and ketose 3-epimerase activity after 24-hour culturing were measured.

The results are shown in Fig. 2. It has been found that the production amount of the enzyme was high in the inorganic salt medium exhibiting the lowest growth out of the investigated media.

### <Experiment 3: Preparation of crude enzyme>

Cells were collected from the culture liquid by centrifugal separation. The cells thus collected were washed with a 10 mM tris-HCl buffer (pH 7.5). Then, after the cells were suspended in 10 ml of 10 mM tris-HCl buffer (pH 7.5), cell disruption was performed by an ultrasonic homogenizer (product of Japan Emerson) while cooling the resulting cell suspension in ice water. The disruption product was centrifuged at 12000 rpm for 20 minutes to obtain a centrifugation supernatant as a crude enzyme.

### <Experiment 4: Measurement of activity of crude enzyme>

The activity of an enzyme ketose 3-epimerase before purification was obtained by measuring the preparation amount of D-fructose with D-allulose as a substrate. More specifically, an enzymatic reaction solution composed of a 50 mM tris-HCl buffer (pH 7.5), 0.1 M D-allulose, an enzyme solution, and 2 mM magnesium chloride is reacted at 70°C for 10 minutes. The reaction mixture is boiled for 2 minutes to terminate the reaction and the solution composition after the reaction is measured by HPLC. One unit of enzyme activity is defined as an enzyme amount that epimerizes D-allulose to prepare 1 µmol of D-fructose per minute under the above-described conditions. An enzyme unit for epimerization of D-fructose into D-allulose, which is a reverse reaction, is also measured under similar conditions and defined similarly.

### <Experiment 5: Purification of enzyme>

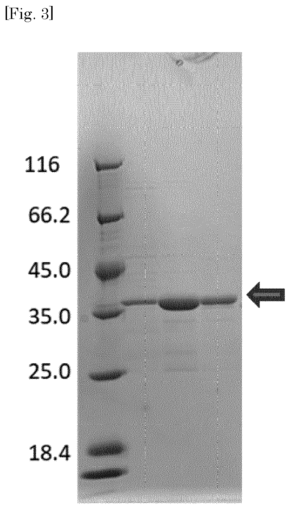

1. Purification by ion exchange chromatography and hydrophobic chromatography
   The crude enzyme solution was purified by ion chromatography. The column used was HiTrapQ HP equilibrated with a buffer (20 mM tris-HCl, pH 7.5). A DUO FLOW system was used and the solution was fractionated into 5 ml fractions at a flow rate of 5 ml/min and a concentration gradient of 1 M NaCl from 0% to 100%. The fraction from which enzyme activity was detected was collected and a purified enzyme by ion exchange chromatographic separation was obtained.
2. The enzyme purified by ion exchange chromatographic separation was purified further by hydrophobic chromatography. The column used was HiTrap PHENYL and ammonium sulfate was added to and dissolved in the enzyme solution to obtain a 2 M solution. The resulting solution was eluted by reducing the concentration of 2 M ammonium sulfate from 100% to 0% at a flow rate of 5 ml/min and fractionated into 5 mL portions. The fractions from-which enzyme activity was detected were collected and dialyzed to remove ammonium sulfate and a purified enzyme by hydrophobic chromatographic separation was obtained.
3. Polyacrylamide gel electrophoresis
   SDS-PAGE (gel concentration: 12.5%) was performed by the conventional method to confirm the purity of the purified enzyme. In Fig. 3, a protein having a standard molecular mass is shown on the left side and the center lane shows the enzyme purified by hydrophobic chromatography. According to the results, a single band is found at about 36 kDa, revealing that the enzyme was purified completely and the subunit of the present enzyme thus purified had a molecular mass of about 36 kDa as determined by SDS-PAGE.

### <Experiment 6: Measurement of physicochemical properties of crude enzyme and purified enzyme>

As a comparison control enzyme, a ketose 3-epimerase derived from Arthrobacter globiformis M30 described in Patent Document 5 was used. The enzyme will hereinafter be called "control enzyme" and will be sometimes referred to as "AgDAE".

For the measurement of the activity of a ketose 3-epimerase as a crude enzyme and a purified enzyme, an enzyme reaction was made by performing an experiment similar to Experiment 4. An enzyme reaction was made for 10 minutes under various conditions and an enzyme amount preparing 1 µmol of D-fructose from D-allulose per minute was defined as 1 unit. After the reaction, the reaction mixture was put in a boiling liquid for 3 minutes to terminate the reaction. After deionization treatment, the D-fructose thus prepared was measured by HPLC analysis.

### 1. Optimum reaction temperature (crude enzyme of wild strain)

Reaction conditions are shown in Table 1 under which the reaction was made at various temperatures within from 50 to 80°C to find an optimum temperature. A temperature range from 60°C to 80°C is preferred for the crude enzyme and Fig. 1 showing the measurement results of a reaction temperature and a relative activity has revealed that the optimum temperature of the crude enzyme of the present enzyme is 70°C when Mg²⁺ ion is added.

**[Table 1]**

| Reaction conditions | |
|---|---|
| Crude enzyme solution | |
| D-allulose | Final concentration: 0.1 M |
| Magnesium chloride | Final concentration: 2 mM |
| Tris-HCl buffer (pH 7.5) | Final concentration: 50 mM |
| Reaction temperature | 50 to 80°C |
| Reaction time | 10 min |

The optimum temperature of a crude enzyme of the control enzyme is also 70°C but, in particular, the relative activity (%) of the crude enzyme of the present enzyme supposing that the activity at 70°C at which it was highest as shown in Fig. 1 is 100 is 77.6% at 80°C and 80.3% at 60°C, while that of the crude enzyme of the control enzyme is 58.7% at 80°C and 96.8% at 60°C, suggesting that the present enzyme particularly has a characteristic of maintaining its activity at a temperature higher than the optimum temperature.

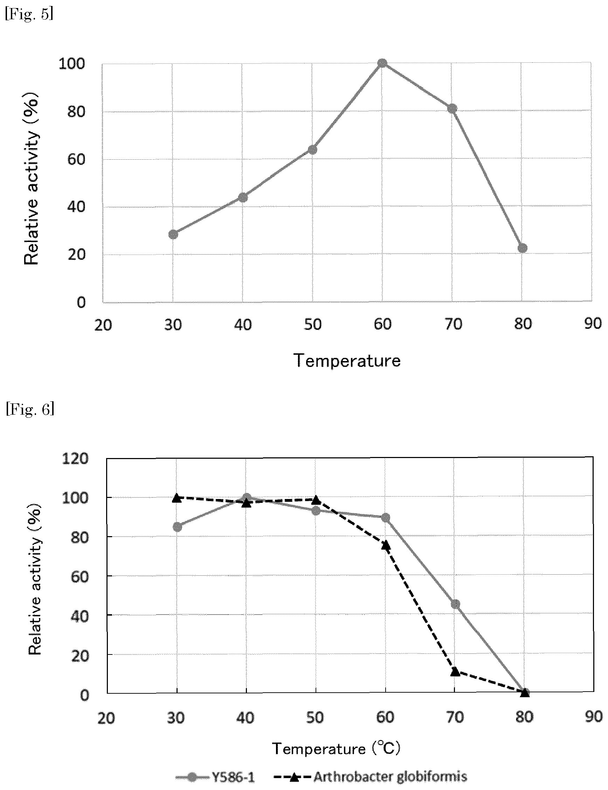

The optimum reaction temperature of the present purified enzyme (purified enzyme of a wild strain) was found under similar conditions, resulting in 60°C as shown in Fig. 5. Fig. 6 shows the residual activity of the present purified enzyme and the control enzyme after incubation at various temperatures for 10 minutes. With respect to the temperature stability after the incubation for 10 minutes, a decrease in the relative activity of the present enzyme at 60°C is less than that of the control enzyme and at 70°C, a difference in decrease of the relative activity increases, which has revealed that compared with the control enzyme, the present enzyme is a more stable enzyme at high temperature.

### 2. Ketose 3-epimerase activity

By using the crude enzyme solution prepared in Experiment 3, a 50 mM tris-HCl buffer (pH 7.5), 0.1 M D-allulose, and 2 mM magnesium chloride, a reaction is conducted for 10 minutes at 70°C. The reaction mixture is then boiled for 2 minutes to terminate the reaction and subjected to HPLC to measure the composition of the reaction mixture. One unit (U) of enzyme activity is the amount of an enzyme that epimerizes D-allulose and prepares 1 µmol of D-fructose per minute under the above conditions. The reaction conditions are shown in Table 2.

**[Table 2]**

| Reaction conditions | |
|---|---|
| Crude enzyme solution | |
| D-allulose | Final concentration: 0.1 M |
| Magnesium chloride | Final concentration: 2 mM |
| Tris-HCl buffer (pH 7.5) | Final concentration: 50 mM |
| Reaction temperature | 70°C |
| Reaction time | 10 min |

The total activity of the crude enzyme obtained from 100 mL of the culture liquid was 2.694 U, while the total activity of the crude enzyme of the control enzyme obtained from 100 mL of the culture liquid was 0.847 U. The results have revealed that the activity of the present enzyme is about 3 times higher than that of the control enzyme.

### 3. Optimum reaction pH (purified enzyme of wild strain)

For the measurement, a reaction was made at 70°C for 10 minutes by using D-allulose as a substrate and using various buffers having from pH 4 to 11 and the optimum reaction pH was determined by measuring the resulting D-fructose by HPLC.

The reaction conditions are shown in Table 3. The buffers used are shown in Table 4.

**[Table 3]**

| Reaction conditions | |
|---|---|
| Enzyme solution | |
| D-allulose | Final concentration: 0.1 M |
| Magnesium chloride | Final concentration: 2 mM |
| Buffer (pH 2.2 to 11.0) | Final concentration: 50 mM |
| Reaction temperature | 70°C |
| Reaction time | 10 min |

**[Table 4]**

| Buffer | |
|---|---|
| Glycine-HCl | pH2.2, pH3.0 |
| Citrate | pH3.0, pH4.0 |
| Acetate | pH4.0, pH5.0, pH6.0 |
| Phosphate | pH6.0, pH7.0, pH8.0 |
| Tris-HCl | pH7.5, pH8.0, pH9.0 |
| Glycine-NaOH | pH9.0, pH10.0, pH11.0 |

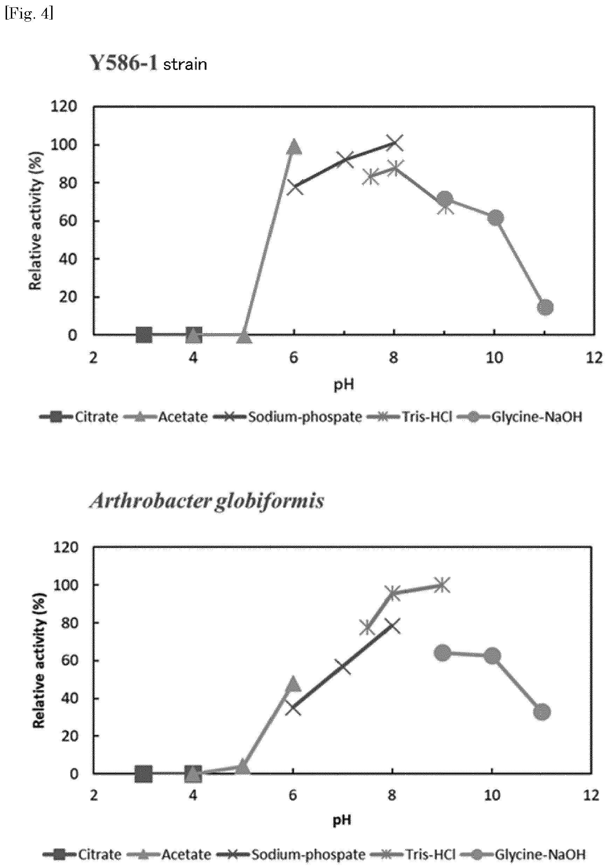

The results are shown in Fig. 4. The optimum reaction pH of the purified enzyme of the present enzyme is from 6 to 8, while the optimum reaction pH of the control enzyme is from 7.5 to 9 and is on the alkaline side. The optimum reaction pH of the present enzyme is over a wider range and the enzyme shows a high activity under more acidic side.

### 4. Thermal stability (crude enzyme of wild strain)

Thermal stability of the enzyme was investigated by measuring the activity of the enzyme after incubation at 60°C for one hour and finding its relative activity supposing that the activity at 60°C under the reaction conditions (10 minutes) used for determining the optimum temperature in the above 2 shown in Table 2 was 100. As heat treatment conditions, the crude enzyme solution, a buffer (50 mM tris-HCl, pH 7.5), and 2 mM MgCl₂ were used to give a total amount of 200 µl and after retaining at 60°C for one hour, the reaction mixture was cooled for 10 minutes in ice water. The residual enzyme activity was then measured by the conventional method.

As shown in Table 7, the residual activity after incubation at 60°C for 1 hour is 49.4% for the present enzyme, while it is 30.8% for the control enzyme, which has revealed that the present enzyme has high thermal stability.

### 5. Substrate specificity of ketose 3-epimerase (purified enzyme of wild strain)

The substrate specificity of the present enzyme after purification was investigated using 8 kinds of ketohexoses. With respect to an enzyme reaction composition, each substrate had a final concentration of 0.1 M and a phosphate buffer had a final concentration of 50 mM and pH 8.0 as shown below in Table 5. After a reaction at 70°C for 10 minutes, the reaction mixture was analyzed by HPLC to measure a ketose produced by epimerization of each sugar.

Supposing that the epimerization activity for D-allulose is 100, the activity for each ketose is shown as a relative activity.

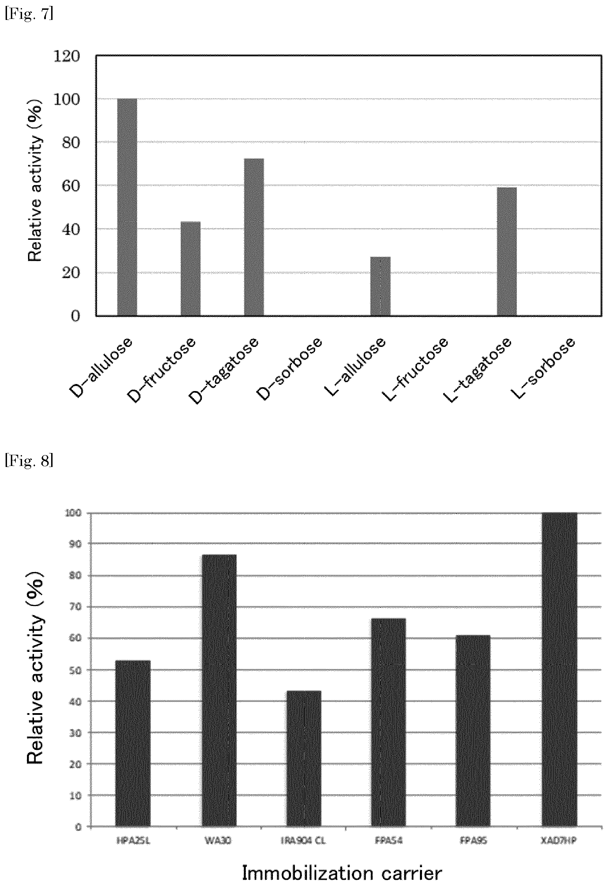

The activity for D-fructose, D-allulose, D-sorbose, D-tagatose, L-fructose, L-allulose, L-sorbose, and L-tagatose used as a substrate is shown in Table 6 and Fig 7 as a relative activity.

**[Table 5]**

| Reaction conditions | |
|---|---|
| Enzyme solution | |
| Substrate | Final concentration: 0.1 M |
| Magnesium chloride | Final concentration: 2 mM |
| Phosphate buffer (pH 8.0) | Final concentration: 50 mM |
| Reaction temperature | 70°C |
| Reaction time | 10 min |

**[Table 6]**

| Substrate | Relative activity (%) |
|---|---|
| D-allulose | 100.0 |
| D-fructose | 43.4 |
| D-tagatose | 72.2 |
| D-sorbose | trace |
| L-allulose | 27.1 |
| L-fructose | trace |
| L-tagatose | 59.2 |
| L-sorbose | trace |

The present enzyme (purified enzyme of wild strain) has the highest activity for D-allulose among ketohexoses and the next highest one is for D-tagatose. Judging from such substrate specificity, compared with another ketose 3-epimerase, the enzyme can be called D-allulose 3-epimerase (DAE) and it shows obviously different specificity from D-tagatose 3-epimerase (DTE) derived from Pseudomonas chicory

### 6. Heat resistance (thermal stability) experiment (crude recombinant enzyme)

### (1) Ketose 3-epimerase activity ratio (T70/T50) at a reaction temperature of 70°C to 50°C

Heat resistance of the present enzyme (crude recombinant enzyme) and the crude enzyme of the control enzyme was investigated by measuring an activity at 70°C and an activity at 50°C under the reaction conditions (10 minutes) used for determining the optimum temperature in the above 2 shown in Table 2 and finding their ratio (T70/T50).

The results are shown in Table 7. The present enzyme (crude recombinant enzyme) has a T70/T50 ratio of 1.78, while the control enzyme has that of 1.54, revealing that the present enzyme (crude recombinant enzyme) has a relatively high activity at high temperature.

### (2) Residual activity after incubation at 60°C for one hour

The activity of each of the present enzyme (crude recombinant enzyme) and the crude enzyme of the control enzyme after incubation at 60°C for 1 hour was measured and a relative activity of it was determined supposing that the activity at 60°C under the reaction conditions (10 minutes) for determining the optimum temperature in the above 2 shown in Table 2 is 100. The relative activity was used as a heat resistance index of the enzyme. As heat treatment conditions, the crude enzyme solution, a buffer (50 mM tris-HCl, pH 7.5), and 2 mM MgCl₂ were used to give a total amount of 200 µl and after retaining at 60°C for one hour, the reaction mixture was cooled for 10 minutes in ice water. The residual enzyme activity was measured by the conventional method.

The results are shown in Table 7. The residual activity after incubation at 60°C for 1 hour is 49.4 for the present enzyme (crude recombinant enzyme), while it is 30.8 for the control enzyme, which has revealed that the present enzyme (crude recombinant enzyme) has high thermal stability at 60°C.

**[Table 7]**

| | | Present enzyme | Control enzyme |
|---|---|---|---|
| Optimum enzyme reaction temperature | | 70°C | 60°C |
| Total activity per 100 mL of culture liquid (optimum reaction temperature) | | 979.3 U | 595.5 U |
| Investigation of optimum temperature | Relative activity at 60°C (%) | 76.4 | 100.0 |
| | Relative activity at 70°C (%) | 100.0 | 94.9 |
| | Relative activity at 80°C (%) | 59.4 | 50.6 |
| T70/T50 | | **1.78** | **1.54** |
| Residual activity after incubation at 60°C for 1 hour | | **49.4** | **30.8** |

### <Experiment 7: Preparation of immobilized enzyme>

The following 1 to 5 are immobilization experiments relating to the immobilization using a crude enzyme of wild strain, and the Y586-1 strain cells cultured and collected as in Experiment 2 were used.

### 1. Acquisition of crude enzyme by ultrasonic treatment of cells

Cells (10 g, wet weight) were suspended in 10 ml of a 50 mM tris-HCl buffer (pH 7.5). The cells were then disrupted using an ultrasonic homogenizer while the resulting cell suspension was cooled in ice water. The disrupted product was centrifuged at 12000 rpm for 30 minutes and the centrifugation supernatant thus obtained was used as a crude enzyme solution.

### 2. Immobilization of crude enzyme

The crude enzyme solution obtained above was added to an ion exchange resin or a synthetic adsorbent washed sufficiently with pure water, swollen therewith, and then equilibrated with a 50 mM tris-HCl buffer (pH 7.5) and a crude enzyme protein was bound to it at 4°C for 24 hours. Then, after washing with a 50 mM tris-HCl buffer (pH 7.5), an immobilized enzyme was obtained.

As a preliminary experiment, an immobilization experiment was performed using strongly basic anion exchange resins (SA10A, SA11A, SA12A, NSA100, SA20A, PA306S, PA308, PA312, PA316, HPA25L, PA408, PA412, and PA418, each product of Mitsubishi Chemical, and IRA904CL, product of Organo), weakly basic anion exchange resins (WA10, WA20, WA21J, and WA30, each product of Mitsubishi Chemical, and FPA54, FPA60CL, and FPA95, each product of Organo), and synthetic adsorbents (XAD7HP and XAD118ON, each product of Organo). The strongly base anion exchange resins HPA25L and IRA904CL, the weakly basic anion exchange resins WA30, FPA54, and FPA95, and the synthetic adsorbent XAD7HP, each allowing the immobilized enzyme, which had been obtained by the adsorption of the crude enzyme solution to the ion exchange resin or synthetic adsorbent, to have an activity expression rate of 30% or more, were used for subsequent experiments.

### 3. Method of measuring the enzyme activity of immobilized enzyme

The enzyme activity of an immobilized enzyme was measured by measuring the amount of D-fructose prepared by carrying out an enzyme reaction with D-allulose as a substrate.

An immobilized enzyme resin (100 mg), a tris-HCl buffer (pH 7.5) (final concentration: 50 mM), and a D-allulose (final concentration: 100 mM) solution (400 µ l) were used as a reaction solution composition. After the reaction solution composition was reacted for from 10 to 30 minutes in a constant-temperature water bath of 30°C, the reaction mixture was boiled at 100°C for 2 minutes to immediately terminate the reaction. The solution after the reaction was cooled to room temperature, desalted with an ion exchange resin (mixed resin of 200CT and IRA67) (each, product of Organo)), and then subjected to filter treatment to obtain an analysis sample. The analysis was conducted by measuring the peak area of the resulting D-fructose by high-performance liquid chromatography (column: GL-C611 (Hitachi), temperature: 60°C, eluent: 0.1 mM NaOH, flow rate: 1.0 ml/min, detector: RID-20A (Shimadzu)).

### 4. Comparison in enzyme activity among carriers used for enzyme immobilization

To find the influence of a carrier to be used for enzyme immobilization, the enzyme activities of the present enzyme immobilized on the following carriers: HPA25L, WA30, IRA904CL, FPA54, FPA95, and XAD7HP were compared. It is to be noted that a relative activity was determined by calculation supposing that the activity of a sample having the largest peak area among comparison samples was 100.

The relative activity of each of the immobilized enzymes is shown in Fig. 8. The activity of the immobilized enzyme prepared using the synthetic adsorbent XAD7HP was highest. With an increase in the amount of the crude enzyme to be immobilized, a relative activity on any resin became higher.

### 5. Comparison in enzyme activity among immobilized enzymes, depending on temperature

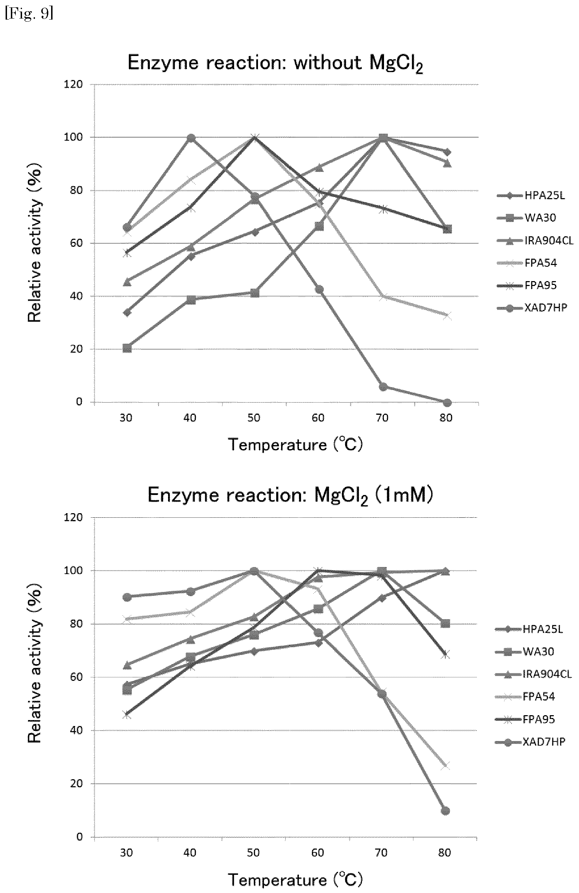

A reaction was conducted at various temperatures of from 30 to 80°C and the relative activity of various immobilized enzymes was measured for each carrier. The results are shown in Fig. 9.

Irrespective of whether Mg²⁺ ion was added or not, the immobilized enzyme using the synthetic adsorbent XAD7HP had a high relative activity at normal temperature and the immobilized enzyme using the anionic exchange resin HPA25L, IRA904CL, or WA30 had a high relative activity at high temperature, suggesting that an immobilization carrier should be selected, depending on the reaction temperature.

### <Experiment 8: Cloning of DNA encoding an enzyme and preparation of a recombinant vector and transformed host cells containing it>

A DNA encoding a ketose 3-epimerase enzyme was cloned from an Arthrobacter histidinolovorans Y586-1 strain, followed by preparation of an autonomously replicable recombinant DNA, determination of the base sequence of the DNA encoding the enzyme, and preparation of a transformed microorganism.

### <Experiment 8-1: Determination of complete base sequence of chromosomal DNA>

It was impossible to isolate the present ketose 3-epimerase enzyme by using the PCR amplification method or existing protein data base, unlike the existing enzymes analogous thereto. The complete genome sequence of the Y586-1 strain was therefore determined and data base of a protein group encoded by all ORFs in the genome sequence was constructed. Macrogen Japan was asked to conduct next-generation sequence analysis using PacBio-RSII/Sequel, with the Y586-1 strain cultured cells as experiment cells.

As a result, two contigs with the base number of 4,948,442 bp and 188,677 bp were obtained, respectively. Since the total base of these two contigs is about 5.1 Mb, it is presumed that they can cover all the genome sequences of the Arthrobacter histidinolovorans Y586-1 strain.

### <Experiment 8-2: Construction of protein data base>

Based on the DNA sequence of about 5.1 Mb thus obtained, 4,734 ORFs were presumed using the Prokka program and the amino acid sequence of each of the ORFs was presumed. The 4,734 amino acid sequences thus presumed were used as a protein data base of the Y586-1 strain.

### <Experiment 8-3: Identification of protein with ketose 3-epimerase activity>

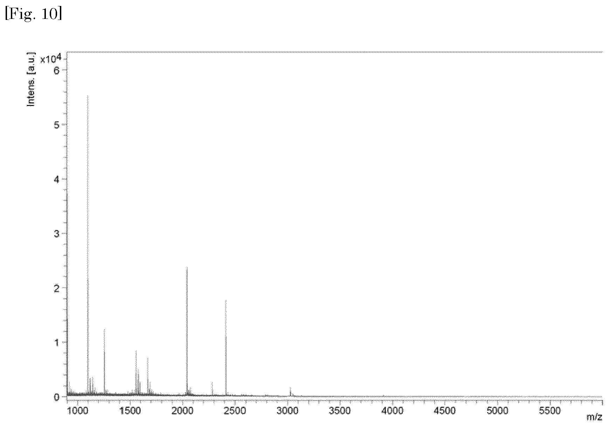

The above-described protein data base was registered on MASCOT server (product of Matrix Science), a protein identification system, and a protein found to have a ketose 3-epimerase activity was identified. As a sample for the experiment, a 36 kDa band of SDS-PAGE in the paragraph [0057] was used and after reduction and alkylation treatments, a trypsin-digested fragment was provided for MALDI-TOF-MS analysis (Fig. 10).

As a result, it was found to have 87% homology to the underlined amino acid sequence in the amino acid sequence of the following Sequence 1 (SEQ ID NO: 1 of Sequence Listing) composed of 289 amino acids, strongly suggesting that the present protein composed of the amino acids of SEQ ID NO: 1 was ketose 3-epimerase of the Y586-1 strain.
[Sequence 1] (SEQ ID NO: 1)

MKIGCHGLVWTGR FDAEGIRYSVQK
TKEAGFDLIEFPLMDPFSFDVATAKSALAEHGLTASASLGLSEATDVSSEDPAIVKAGEE
LLNRALDVLAELGATDFCGVIYSAMKKYMEPATEQGLSNSK AAVGRVVDR
AAGLGINVSLEVVNRYETNVLNTGRQALSYLSDVKRPNLGVHLDTYHMNIEESDMFSP
VLDTAEALKYVHIGESHRGYLGTGSVDFDNFFKALGRIGYDGPVVFESFSSAVVAPDLS
RMLGIWRNLWADNEELGAHANAFIRDK LTAIKTIELH

(Sequence 1: an amino acid sequence identified using the protein data base of the Y586-1 strain. The underlined amino acid sequence was identical in peak obtained by MALDI-TOF-MS)

### <Experiment 8-3: Isolation of ketose 3-epimerase enzyme gene>

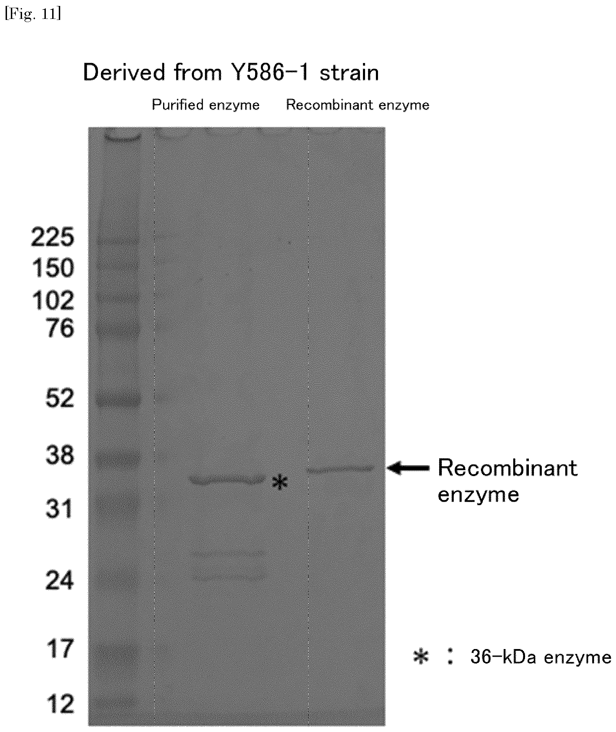

The DNA sequence (SEQ ID NO: 2 of Sequence Listing) of a gene identified by the amino acid sequence was synthesized and inserted in a pQE60 vector (Qiagen) and an Escherichia coli for expression was transformed with it. The Escherichia coli expression system thus constructed was used to confirm an induced enzyme. As shown in the results of SDS-PAGE in Fig. 11, an induced protein having RSHHHHHH added at the C terminal thereof was found in a soluble fragment. Since the recombinant enzyme has an RSHHHHHH amino acid sequence added at the C terminal thereof, it is larger by about 1 kDa than the purified enzyme of the Y586-1 strain. In addition, the ketose 3-epimerase activity of the band was confirmed.
[Sequence 2] (SEQ ID NO: 2)

ATGAAAATTGGCTGCCACGGCCTGGTATGGACCGGCCGTTTCGACGCTGAAGGCAT
CCGCTACTCCGTCCAGAAGACCAAGGAAGCCGGGTTTGATCTGATCGAATTCCCGCT
CATGGATCCCTTCTCCTTCGATGTGGCCACGGCAAAGTCGGCACTCGCAGAGCATGG
TTTGACCGCCTCCGCATCATTGGGACTTTCCGAAGCTACGGATGTCAGCAGTGAAGA
TCCGGCCATTGTGAAAGCGGGGGAGGAGTTGCTCAACCGTGCCCTTGATGTTCTTGC
GGAGCTGGGTGCCACCGACTTCTGCGGCGTGATCTACAGTGCGATGAAGAAGTACA
TGGAGCCGGCCACCGAGCAAGGCTTGAGCAACAGCAAGGCCGCTGTCGGGAGGGTG
GTCGACAGGGCTGCTGGCCTGGGCATCAACGTCTCGCTTGAGGTCGTGAACCGGTAT
GAAACCAACGTGCTGAACACCGGGCGGCAGGCTCTGTCCTACCTCTCTGATGTCAAG
CGGCCGAATCTAGGCGTTCATTTGGATACTTACCACATGAACATCGAGGAATCGGAC

ATGTTCTCTCCGGTGCTGGACACCGCCGAGGCTCTTAAGTACGTGCACATTGGCGAG
AGCCACCGAGGTTACCTCGGCACCGGGAGCGTCGATTTCGACAACTTCTTCAAGGCT
CTTGGCCGCATCGGCTACGACGGACCGGTCGTCTTCGAATCATTCTCCTCCGCGGTC
GTGGCCCCGGATCTGAGCCGCATGCTTGGCATCTGGCGCAACCTGTGGGCGGACAA
CGAAGAACTCGGCGCCCACGCCAACGCCTTCATACGCGACAAACTCACGGCCATCA
AGACCATCGAACTGCACTAA

(Sequence 2: DNA sequence identified using the protein data base of the Y586-1 strain. The underlined base corresponding to a stop codon was removed at the time of cloning.)

### <Experiment 9: Mutant having 70% or more amino acid sequence identity to the present enzyme and its enzyme activity>

A mutant having the following Sequence 3 (SEQ ID NO: 3 of Sequence Listing) and a 78.5% amino acid sequence identity to the amino acid sequence of Sequence 1 and a mutant having the following Sequence 4 (SEQ ID NO: 4 of Sequence Listing) and 76.8% amino acid sequence identity were prepared and the enzyme activity and heat resistance of the crude enzymes were measured in a manner similar to that of 2 and 6 of Experiment 6.
[Sequence 3] (SEQ ID NO: 3) (The underlined are amino acids different from those of SEQ ID NO: 1) 
MNIGCHGLVWTGTFDADGIRLAVEKTKQAGFDLIEFPLMDPFSFDVAAAQAALAEHGL
AVSASLGLSDETDITSSDPAVVAAGEALLLRAVDVLAELGGTHFCGVIYSAMKKYMDP
VTPEGLASSRRTIARVADHAAERGVSVSLEVVNRYETNVLNTARQALAYLGEVDRPNL
GIHLDTYHMNIEESDMFAPVLDAAPALRYVHIGESHRGYLGTGTVDFDTFFKALGRIGY
DGPIVFESFSSAVVAPDLSRMLGIWRNLWTDNDELGAHANAYIRDKLVAVDSIRLH
[Sequence 4] (SEQ ID NO: 4) (The underlined are amino acids different from those of SEQ ID NO: 1)

MNIGCHGLVWTGNFDAEGIRLAVHKTREAGFDLIEFPLMDPFTFDVSVAKDALEEYDIA
VSASLGLSEETDVTSADPAVAAAGEALLIKAVDVLADLGGEHFCGVIYSAMKKYMEPV
TPEGLASSKAAIARVADHAAARGVSVSLEVVNRYETNVLNTARQALAYIAEVDRPNLGI
HIDTYHMNIEESDMFTPVLDAAPALRYVHIGESHRGYLGTGTVDFDTFFKALGRIGYNG
PIVFESFSSAVVAPDLSRMLGIWRNLWNDNDELGAHANAYIRDKLVAVDSIRLH

It was presumed that the active site residues of the present enzyme were amino acids at positions 6, 63, 110, 152, 179, 182, 205, 211, and 240 and residues at the presumed metal ion binding site were amino acids at positions 146, 177, 205, and 240 so that amino acid substitution in the vicinity of those amino acids was not performed.

The results of crude recombinant enzymes are shown below in Table 8.

**[Table 8]**

| | | Present enzyme | Mutant enzyme Sequence information 3 | Mutant enzyme Sequence information 4 |
|---|---|---|---|---|
| Identity to Y586DAE1 (%) | | 100.0 | 78.5 | 76.8 |
| Culture temperature, induction temperature | | 30°C | 30°C | 30°C |
| Optimum enzyme reaction temperature | | 70°C | 70°C | 70°C |
| Total activity per 100 mL of culture liquid (optimum reaction temperature) | | 979.3 U | 1201 U | 500.0 U |
| Investigation of optimum temperature | Relative activity at 60°C (%) | 76.4 | 75.8 | 88.5 |
| | Relative activity at 70°C (%) | 100.0 | 100.0 | 100.0 |
| | Relative activity at 80°C (%) | 59.4 | 31.2 | 67.3 |
| T70/T50 | | **1.78** | **2.11** | **2.14** |
| Residual activity after incubation at 60°C for 1 hour | | **49.4** | **38.2** | **44.7** |

In Table 8, the term "Y586DAE1" means D-allulose 3-epimerase belonging to ketose 3-epimerase derived from the Y586-1 strain, which will equally apply hereinafter.

It has been confirmed that as shown in Table 8, even a mutant enzyme having about 78% or 77% amino acid identity has rather a high T70/T50 ratio, which is an index of heat resistance as a useful property, while maintaining its enzyme activity and has no change in properties as an enzyme, insofar as amino acid substitution is conducted at a site except for the vicinity of the active site · the presumed metal ion binding site.

### <Experiment 10: Preparation of amino acid substituted mutant of the present enzyme and its enzyme activity>

From comparison between the amino acid sequence of the ketose 3-epimerase of the present invention represented by SEQ ID NO: 1 and the amino acid sequence of the control enzyme (ketose 3-epimerase derived from Arthrobacter globiformis) (Patent Document 5), it was presumed that the site different in amino acid residue was less involved in the ketose 3-epimerase activity. Various ketose 3-epimerase mutants were therefore prepared by changing the amino acids at the different site of SEQ ID NO: 1 by base substitution using site-directed mutagenesis operation and from them, mutants having a site capable of providing amino acid substituted mutants having higher heat resistance than the wild type were selected.

First, a single-amino-acid-substituted mutant was prepared by site-directed mutagenesis. PCR primers for both DNA strands having a base sequence obtained by adding base substitution to one or two base pairs to cause amino acid substitution at the intended mutagenesis site were prepared and a PCR reaction was performed using a plasmid DNA to be subjected to mutagenesis and the above primers. The plasmid DNA was extracted from the clone obtained by transforming the PCR fragment thus obtained into host Escherichia coli and introduction of intended mutation was confirmed by Sequence analysis. A multiple substituted mutant was prepared by repeating the above-described method.

The T70/T50 and the residual activity after incubation at 60°C for 1 hour, each of the crude enzymes of the single-amino-acid-substituted mutants obtained by recombination, were measured. Of these, mutants higher than the control enzyme in either one of the T70/50 or the residual activity after incubation at 60°C for one hour were selected. They were amino acid substituents at 18 sites shown in Table 9.

**[Table 9]**

| No | Mutated amino acid number |
|---|---|
| 1 | H56 |
| 2 | T59 |
| 3 | A60 |
| 4 | P77 |
| 5 | L94 |
| 6 | L97 |
| 7 | D101 |
| 8 | A109 |
| 9 | A128 |
| 10 | V129 |
| 11 | V132 |
| 12 | S144 |
| 13 | S167 |
| 14 | P172 |
| 15 | E199 |
| 16 | A200 |
| 17 | K202 |
| 18 | S218 |

Next, arbitrary double to undecuple mutants were prepared by simultaneously substituting from 2 to 11 amino acids at the above 18 sites and the resulting recombinant mutant crude enzymes were measured for the T70/T50 and the residual activity after incubation at 60°C for 1 hour. The results are shown in Tables 10 and 11.

Among 37 kinds of the site-specific mutant enzymes, 30 kinds had a T70/T50 ratio more than that of the control enzyme AgDAE (1.54), in which 20 kinds of the mutant enzymes among 37 kinds had a ratio more than that of the parent-strain wild-type recombinant enzyme Y586-1 strain DAE (1.78) (Table 10).

Among 37 kinds of the site-specific mutant enzymes, 37 kinds had a residual activity, after incubation at 60°C for 1 hour, more than that of the control enzyme AgDAE (30.8%), in which 36 kinds of the mutant enzymes among 37 kinds had the residual activity more than that of the parent-strain wild-type recombinant enzyme Y586-1 strain DAE (49.4%) (Table 11).

The above-described results have revealed that the ketose 3-epimerase of the present invention is an excellent and useful enzyme capable of providing a number of mutants having higher thermal stability when subjected to site-directed mutagenesis.

**[Table 10]**

| Ketose 3-epimerase mutant | Mutation site | T70/T50 |
|---|---|---|
| 1 | T59SA60VD101EV129IA200VS218N | 2.37 |
| 2 | A200VS218N | 2.31 |
| 3 | D101EV129IA200V | 2.11 |
| 4 | T59SA60VA200VS218N | 2.08 |
| 5 | T59SA60VV129IA200V | 2.07 |
| 6 | S218N | 2.05 |
| 7 | T59SA60VA200V | 2.03 |
| 8 | V129IA200VS218N | 2.03 |
| 9 | T59SA60V | 2.01 |
| 10 | T59SA60VS218N | 1.98 |
| 11 | V129I | 1.95 |
| 12 | V129IS218N | 1.94 |
| 13 | V129IA200V | 1.94 |
| 14 | A200V | 1.91 |
| 15 | T59SA60VD101EA200V | 1.90 |
| 16 | T59SA60VD101EV129I | 1.89 |
| 17 | D101EA200V | 1.89 |
| 18 | D101E | 1.85 |
| 19 | T59SA60VV129IA200VS218N | 1.85 |
| 20 | T59SA60VV129I | 1.79 |
| | Y586-1_DAE (wild type) | 1.78 |
| 21 | T59SA60VD101E | 1.70 |
| 22 | T59SA60VP77SL94VD101EA109SV129IS144AS167QA200VS218N | 1.70 |
| 23 | P172G | 1.69 |
| 24 | S167Q | 1.68 |
| 25 | K202R | 1.66 |
| 26 | L97M | 1.65 |
| 27 | D101EV129I | 1.64 |
| 28 | D101EV129IA200VS218N | 1.62 |
| 29 | H56Y | 1.58 |
| 30 | S144A | 1.57 |
| | Ag_DAE | 1.54 |
| 31 | E199D | 1.54 |
| 32 | P77S | 1.53 |
| 33 | T59SA60VD101EA200VS218N | 1.46 |
| 34 | A128V | 1.38 |
| 35 | V132I | 1.34 |
| 36 | L94V | 1.30 |
| 37 | A109S | 1.07 |

**[Table 11]**

| Ketose 3-epimerase mutant | Mutation site | Residual activity (%) |
|---|---|---|
| 4 | T59SA60VA200VS218N | 123.2 |
| 8 | V129IA200VS218N | 115.3 |
| 22 | T59SA60VP77SL94VD101EA109SV129IS144AS167QA200VS218N | 113.5 |
| 28 | D101EV129IA200VS218N | 112.7 |
| 6 | S218N | 101.2 |
| 3 | D101EV129IA200V | 94.5 |
| 19 | T59SA60VV129IA200VS218N | 91.2 |
| 33 | T59SA60VD101EA200VS218N | 91.0 |
| 12 | V129IS218N | 90.5 |
| 15 | T59SA60VD101EA200V | 89.3 |
| 5 | T59SA60VV129IA200V | 85.1 |
| 13 | V129IA200V | 83.7 |
| 2 | A200VS218N | 83.3 |
| 7 | T59SA60VA200V | 83.1 |
| 10 | T59SA60VS218N | 79.5 |
| 14 | A200V | 79.1 |
| 1 | T59SA60VD101EV129IA200VS218N | 74.1 |
| 18 | D101E | 72.1 |
| 35 | V132I | 69.2 |
| 26 | L97M | 68.9 |
| 11 | V129I | 68.6 |
| 30 | S144A | 66.9 |
| 23 | P172G | 65.8 |
| 9 | T59SA60V | 65.6 |
| 37 | A109S | 63.3 |
| 17 | D101EA200V | 62.3 |
| 27 | D101EV129I | 61.9 |
| 21 | T59SA60VD101E | 59.4 |
| 31 | E199D | 58.1 |
| 34 | A128V | 57.2 |
| 36 | L94V | 57.0 |
| 29 | H56Y | 56.8 |
| 24 | S167Q | 55.4 |
| 16 | T59SA60VD101EV129I | 54.0 |
| 20 | T59SA60VV129I | 52.1 |
| 25 | K202R | 51.4 |
| | Y586-1_DAE (wild type) | 49.4 |
| 32 | P77S | 39.9 |
| | Ag_DAE | 30.8 |

### Industrial Applicability

By allowing the ketose 3-epimerase of the present invention to act on a free D- or L-ketose or D- or L-ketopentose and epimerizing position 3 of these ketoses, a D- or L-ketose or D- or L-ketose corresponding thereto is prepared easily. This reaction paves the way for the mass production of various ketoses, particularly, D-allulose using D-fructose as a raw material.

In particular, compared with the conventional ketose 3-epimerase, the enzyme of the present invention has a total activity, which can be obtained from a culture liquid, about 3 times higher than that of a control enzyme and this enables the mass production of D-allulose.

In addition, the enzyme of the present invention has an optimum temperature as high as 70°C and is therefore a highly heat-resistant enzyme so that a reaction in the vicinity of the optimum temperature of from 60 to 70°C brings about a higher production yield of D-allulose.

Further, an immobilized enzyme having high activity can be obtained by immobilizing the ketose 3-epimerase of the present invention by various immobilization methods. By using an immobilized enzyme, a large amount of an epimerization reaction can be performed continuously. Since the enzyme can be industrially immobilized, intended mass production of a ketose can be achieved.

Further, many of amino acid substituted mutants of the ketose 3-epimerase of the present invention have higher thermal stability than the original enzyme before mutation.

Accordingly, the establishment of the ketose 3-epimerase of the present invention and a production method thereof have a remarkably great industrial significance not only in the sugar industry but also in food, cosmetic, and pharmaceutical industries related thereto.

## Claims

1. A ketose 3-epimerase derived from a microorganism belonging to the genus Arthrobacter, having a molecular mass of 36 kDa as measured by SDS-PAGE, and having the following substrate specificities (A) and (B):
(A) having an activity of epimerizing position 3 of a D- or L-ketose and preparing a D- or L-ketose corresponding thereto, and
(B) having the highest epimerization activity on position 3 of D-allulose among D- or L-ketohexoses.

2. The ketose 3-epimerase according to Claim 1, having the following physicochemical properties (C) and (D):
(C) having an optimum reaction pH of from 6 to 8, and
(D) having an optimum reaction temperature of 60°C.

3. The ketose 3-epimerase according to Claim 1 or 2, wherein the substrate specificity is in the order of D-allulose, D-tagatose, L-tagatose, D-fructose, and L-allulose.

4. The ketose 3-epimerase according to any of Claims 1 to 3, wherein the microorganism belonging to the genus Arthrobacter is Arthrobacter histidinolovorans.

5. The ketose 3-epimerase according to any of Claims 1 to 4, wherein the microorganism belonging to the genus Arthrobacter is an Arthrobacter histidinolovorans Y586-1 strain deposited under an accession number NITE BP-02813 at the Patent Microorganisms Depositary Center.

6. A protein described in the following (a) or (b):
(a) a protein having an amino acid sequence represented by SEQ ID NO: 1, or
(b) a protein having an amino acid sequence having 70% more identity to the amino acid sequence represented by SEQ ID NO: 1, having the following ketose 3-epimerase activities (A) and (B), and having a ketose 3-epimerase activity ratio (T70/T50) of 1.55 or more at a reaction temperature of 70°C to 50°C:
(A) having an activity of epimerizing position 3 of a D- or L-ketose and preparing a D- or L-ketose corresponding thereto, and
(B) having the highest epimerization activity on position 3 of D-allulose among D- or L-ketohexoses.

7. A protein described in the following (a) or (b):
(a) a protein having an amino acid sequence represented by SEQ ID NO: 1, or
(b) a protein having an amino acid sequence having 70% more identity to the amino acid sequence represented by SEQ ID NO: 1, having the following ketose 3-epimerase activities (A) and (B), and having a residual activity of 31% or more after incubation at 60°C for one hour:
(A) having an activity of epimerizing position 3 of a D- or L-ketose and preparing a D- or L-ketose corresponding thereto, and
(B) having the highest epimerization activity on position 3 of D-allulose among D- or L-ketohexoses.

8. A protein comprising an amino acid-substituted mutant of a protein having an amino acid sequence represented by SEQ ID NO: 1, wherein the mutant has amino acid substitution at at least one site selected from H56, T59, A60, P77, L94, L97, D101, A109, A128, V129, V132, S144, S167, P172, E199, A200, K202, and S218 of the amino acid sequence represented by SEQ ID NO: 1, has the following ketose 3-epimerase activities (A) and (B), and has a higher ketose 3-epimerase activity ratio (T70/T50) at a reaction temperature of 70°C to 50°C or has a higher residual activity after incubation at 60°C for one hour than the protein having an amino acid sequence represented by SEQ ID NO: 1:
(A) having an activity of epimerizing position 3 of a D- or L-ketose and preparing a D- or L-ketose corresponding thereto, and
(B) having the highest epimerization activity on position 3 of D-allulose among D- or L-ketohexoses.

9. A DNA encoding the protein as claimed in any of Claims 6 to 8.

10. A DNA comprising a base sequence represented by SEQ ID NO: 2 or a complementary sequence thereof.

11. The DNA according to Claim 9, **characterized in that** the DNA is any of the following (a) to (c):
(a) a DNA having a sequence containing the whole or a part of a base sequence represented by SEQ ID NO: 2 or a complementary sequence thereof,
(b) a DNA that hybridizes with a complementary strand of a DNA having a base sequence represented by SEQ ID NO: 2 under stringent conditions, and
(c) a DNA having 70% or more sequence identity to a DNA having a base sequence represented by SEQ ID NO: 2.

12. A recombinant vector comprising the DNA as claimed in any of Claims 9 to 11.

13. A transformed host cell obtained by transformation by the recombinant vector as claimed in Claim 12.

14. An Arthrobacter histidinolovorans Y586-1 strain that produces the ketose 3-epimerase as claimed in any one of Claims 1 to 3 and is deposited under an accession number NITE BP-02813 at the Patent Microorganisms Depositary Center.

15. An immobilized enzyme, **characterized in that** the ketose 3-epimerase as claimed in any of Claims 1 to 5 has been immobilized on a carrier.

16. An immobilized enzyme, **characterized in that** a ketose 3-epimerase crude enzyme present in a disrupted cell of a microorganism belonging to the genus Arthrobacter that produces the ketose 3-epimerase as claimed in any of Claims 1 to 5 has been immobilized on a carrier.

17. The immobilized enzyme according to Claim 15 or 16, wherein the carrier is an ion exchange resin, an organic polymer carrier, and/or an inorganic carrier.

18. An immobilized protein, **characterized in that** the protein as claimed in any of Claims 6 to 8 has been immobilized on a carrier.

19. The immobilized protein according to Claim 18, wherein the carrier is an ion exchange resin, an organic polymer carrier, and/or an inorganic carrier.

20. A method of producing a ketose 3-epimerase, comprising culturing, in a medium, a microorganism belonging to the genus Arthrobacter that produces the ketose 3-epimerase as claimed in any of Claims 1 to 5, accumulating the ketose 3-epimerase in a microorganism cell, and collecting the resulting ketose 3-epimerase.

21. The method of producing a ketose 3-epimerase according to Claim 20, wherein the medium is a D-allulose-added inorganic salt medium.

22. A method of producing a protein having a ketose 3-epimerase activity, comprising culturing the transformed host cell as claimed in Claim 13 in a medium and collecting a protein having a ketose 3-epimerase activity from a cultured product.

23. A method of producing a ketose, comprising allowing the ketose 3-epimerase as claimed in any of Claims 1 to 5, the protein as claimed in any of Claims 6 to 8, the immobilized enzyme as claimed in any of Claims 15 to 17, or the immobilized protein as claimed in Claim 18 or 19 to act on a solution containing at least one selected from D- or L-ketoses to epimerize position 3 of the ketose and prepare a ketose corresponding thereto and collecting the resulting ketose.


SEQUENCE LISTING

<110>  NATIONAL UNIVERSITY CORPORATION KAGAWA UNIVERSITY

<120>  Novel ketose 3-epimerase

<130>  PCT19347UK

<150>  JP 2018-218287
<151>  2018-11-21

<160>  4

<170>  PatentIn version 3.5

<210>  1
<211>  289
<212>  PRT
<213>  Arthrobacter histidinolovorans

<400>  1

Met Lys Ile Gly Cys His Gly Leu Val Trp Thr Gly Arg Phe Asp Ala
1               5                   10                  15

Glu Gly Ile Arg Tyr Ser Val Gln Lys Thr Lys Glu Ala Gly Phe Asp
            20                  25                  30

Leu Ile Glu Phe Pro Leu Met Asp Pro Phe Ser Phe Asp Val Ala Thr
        35                  40                  45

Ala Lys Ser Ala Leu Ala Glu His Gly Leu Thr Ala Ser Ala Ser Leu
    50                  55                  60

Gly Leu Ser Glu Ala Thr Asp Val Ser Ser Glu Asp Pro Ala Ile Val
65                  70                  75                  80

Lys Ala Gly Glu Glu Leu Leu Asn Arg Ala Leu Asp Val Leu Ala Glu
                85                  90                  95

Leu Gly Ala Thr Asp Phe Cys Gly Val Ile Tyr Ser Ala Met Lys Lys
            100                 105                 110

Tyr Met Glu Pro Ala Thr Glu Gln Gly Leu Ser Asn Ser Lys Ala Ala
        115                 120                 125

Val Gly Arg Val Val Asp Arg Ala Ala Gly Leu Gly Ile Asn Val Ser
    130                 135                 140

Leu Glu Val Val Asn Arg Tyr Glu Thr Asn Val Leu Asn Thr Gly Arg
145                 150                 155                 160

Gln Ala Leu Ser Tyr Leu Ser Asp Val Lys Arg Pro Asn Leu Gly Val

165                 170                 175

His Leu Asp Thr Tyr His Met Asn Ile Glu Glu Ser Asp Met Phe Ser
            180                 185                 190

Pro Val Leu Asp Thr Ala Glu Ala Leu Lys Tyr Val His Ile Gly Glu
        195                 200                 205

Ser His Arg Gly Tyr Leu Gly Thr Gly Ser Val Asp Phe Asp Asn Phe
    210                 215                 220

Phe Lys Ala Leu Gly Arg Ile Gly Tyr Asp Gly Pro Val Val Phe Glu
225                 230                 235                 240

Ser Phe Ser Ser Ala Val Val Ala Pro Asp Leu Ser Arg Met Leu Gly
                245                 250                 255

Ile Trp Arg Asn Leu Trp Ala Asp Asn Glu Glu Leu Gly Ala His Ala
            260                 265                 270

Asn Ala Phe Ile Arg Asp Lys Leu Thr Ala Ile Lys Thr Ile Glu Leu
        275                 280                 285

His

<210>  2
<211>  870
<212>  DNA
<213>  Arthrobacter histidinolovorans

<400>  2
atgaaaattg gctgccacgg cctggtatgg accggccgtt tcgacgctga aggcatccgc      60

tactccgtcc agaagaccaa ggaagccggg tttgatctga tcgaattccc gctcatggat     120

cccttctcct tcgatgtggc cacggcaaag tcggcactcg cagagcatgg tttgaccgcc     180

tccgcatcat tgggactttc cgaagctacg gatgtcagca gtgaagatcc ggccattgtg     240

aaagcggggg aggagttgct caaccgtgcc cttgatgttc ttgcggagct gggtgccacc     300

gacttctgcg gcgtgatcta cagtgcgatg aagaagtaca tggagccggc caccgagcaa     360

ggcttgagca acagcaaggc cgctgtcggg agggtggtcg acagggctgc tggcctgggc     420

atcaacgtct cgcttgaggt cgtgaaccgg tatgaaacca acgtgctgaa caccgggcgg     480

caggctctgt cctacctctc tgatgtcaag cggccgaatc taggcgttca tttggatact     540

taccacatga acatcgagga atcggacatg ttctctccgg tgctggacac cgccgaggct     600

cttaagtacg tgcacattgg cgagagccac cgaggttacc tcggcaccgg gagcgtcgat     660

ttcgacaact tcttcaaggc tcttggccgc atcggctacg acggaccggt cgtcttcgaa 720

tcattctcct ccgcggtcgt ggccccggat ctgagccgca tgcttggcat ctggcgcaac 780

ctgtgggcgg acaacgaaga actcggcgcc cacgccaacg ccttcatacg cgacaaactc 840

acggccatca agaccatcga actgcactaa 870

<210> 3
<211> 289
<212> PRT
<213> Artificial Sequence

<400> 3

Met Asn Ile Gly Cys His Gly Leu Val Trp Thr Gly Thr Phe Asp Ala
1               5                   10                  15

Asp Gly Ile Arg Leu Ala Val Glu Lys Thr Lys Gln Ala Gly Phe Asp
            20                  25                  30

Leu Ile Glu Phe Pro Leu Met Asp Pro Phe Ser Phe Asp Val Ala Ala
        35                  40                  45

Ala Gln Ala Ala Leu Ala Glu His Gly Leu Ala Val Ser Ala Ser Leu
    50                  55                  60

Gly Leu Ser Asp Glu Thr Asp Ile Thr Ser Ser Asp Pro Ala Val Val
65                  70                  75                  80

Ala Ala Gly Glu Ala Leu Leu Leu Arg Ala Val Asp Val Leu Ala Glu
                85                  90                  95

Leu Gly Gly Thr His Phe Cys Gly Val Ile Tyr Ser Ala Met Lys Lys
            100                 105                 110

Tyr Met Asp Pro Val Thr Pro Glu Gly Leu Ala Ser Ser Arg Arg Thr
        115                 120                 125

Ile Ala Arg Val Ala Asp His Ala Ala Glu Arg Gly Val Ser Val Ser
    130                 135                 140

Leu Glu Val Val Asn Arg Tyr Glu Thr Asn Val Leu Asn Thr Ala Arg
145                 150                 155                 160

Gln Ala Leu Ala Tyr Leu Gly Glu Val Asp Arg Pro Asn Leu Gly Ile
                165                 170                 175

His Leu Asp Thr Tyr His Met Asn Ile Glu Glu Ser Asp Met Phe Ala
            180                 185                 190

Pro Val Leu Asp Ala Ala Pro Ala Leu Arg Tyr Val His Ile Gly Glu
        195                 200                 205

Ser His Arg Gly Tyr Leu Gly Thr Gly Thr Val Asp Phe Asp Thr Phe
    210                 215                 220

Phe Lys Ala Leu Gly Arg Ile Gly Tyr Asp Gly Pro Ile Val Phe Glu
225                 230                 235                 240

Ser Phe Ser Ser Ala Val Val Ala Pro Asp Leu Ser Arg Met Leu Gly
                245                 250                 255

Ile Trp Arg Asn Leu Trp Thr Asp Asn Asp Glu Leu Gly Ala His Ala
            260                 265                 270

Asn Ala Tyr Ile Arg Asp Lys Leu Val Ala Val Asp Ser Ile Arg Leu
        275                 280                 285

His

<210>  4
<211>  289
<212>  PRT
<213>  Artificial Sequence

<400>  4

Met Asn Ile Gly Cys His Gly Leu Val Trp Thr Gly Asn Phe Asp Ala
1               5                   10                  15

Glu Gly Ile Arg Leu Ala Val His Lys Thr Arg Glu Ala Gly Phe Asp
            20                  25                  30

Leu Ile Glu Phe Pro Leu Met Asp Pro Phe Thr Phe Asp Val Ser Val
        35                  40                  45

Ala Lys Asp Ala Leu Glu Glu Tyr Asp Ile Ala Val Ser Ala Ser Leu
    50                  55                  60

Gly Leu Ser Glu Glu Thr Asp Val Thr Ser Ala Asp Pro Ala Val Ala
65                  70                  75                  80

Ala Ala Gly Glu Ala Leu Leu Ile Lys Ala Val Asp Val Leu Ala Asp
                85                  90                  95

Leu Gly Gly Glu His Phe Cys Gly Val Ile Tyr Ser Ala Met Lys Lys
            100                 105                 110

Tyr Met Glu Pro Val Thr Pro Glu Gly Leu Ala Ser Ser Lys Ala Ala
        115                 120                 125

Ile Ala Arg Val Ala Asp His Ala Ala Ala Arg Gly Val Ser Val Ser
    130                 135                 140

Leu Glu Val Val Asn Arg Tyr Glu Thr Asn Val Leu Asn Thr Ala Arg
145                 150                 155                 160

Gln Ala Leu Ala Tyr Ile Ala Glu Val Asp Arg Pro Asn Leu Gly Ile
                165                 170                 175

His Ile Asp Thr Tyr His Met Asn Ile Glu Glu Ser Asp Met Phe Thr
            180                 185                 190

Pro Val Leu Asp Ala Ala Pro Ala Leu Arg Tyr Val His Ile Gly Glu
        195                 200                 205

Ser His Arg Gly Tyr Leu Gly Thr Gly Thr Val Asp Phe Asp Thr Phe
    210                 215                 220

Phe Lys Ala Leu Gly Arg Ile Gly Tyr Asn Gly Pro Ile Val Phe Glu
225                 230                 235                 240

Ser Phe Ser Ser Ala Val Val Ala Pro Asp Leu Ser Arg Met Leu Gly
                245                 250                 255

Ile Trp Arg Asn Leu Trp Asn Asp Asn Asp Glu Leu Gly Ala His Ala
            260                 265                 270

Asn Ala Tyr Ile Arg Asp Lys Leu Val Ala Val Asp Ser Ile Arg Leu
        275                 280                 285

His